# EUROPEAN PATENT SPECIFICATION

(11) **EP 0 393 786 B1**
(45) Date of publication and mention of the grant of the patent: **17.01.1996**
(21) Application number: 90200954.7
(22) Date of filing: 18.04.1990
(51) Int. Cl.: C07K 5/02

(54) **New retro-inverse, one- or more bond bearing analogues of thymopentin, a method for synthesizing the same and their employment for the preparation of pharmaceutical**
Retro-Inverso-Peptide, Analoga von Thymopenthin, die eine oder mehrere Bindungen tragen, Verfahren zu deren Herstellung und deren Verwendung zur Zubereitung eines Medikamentes
Rétro-inverso peptides, analogues de la thymopentine ayant une ou plusieurs liaisons, procédés pour les préparer et leur utilisation pour confectionner un médicament

(30) Priority: 21.04.1989 IT 2025789
(43) Date of publication of application: 24.10.1990
(73) Proprietor: SCLAVO S.p.A., I-53100 Siena (IT)
(72) Inventor: Mariotti, Sabina, I-02032 Fara Sabina, Rieti (IT); Sisto, Alessandro, I-00136 Rome (IT); Nencioni, Luciano, I-53036 Poggibonsi, Siena (IT); Villa, Luigi, I-50124 Florence (IT); Verdini, Antonio Silvio, I-0015 Monterotondo, Rome (IT); Pessi, Antonello, I-00199 Rome (IT)
(74) Representative: Gervasi, Gemma, Dr.

(56) References cited:
- EP-A- 0 282 291
- JOURNAL OF THE AMERICAN CHEMICAL SOCIETY, vol. 99, no. 24, 23 November 1977; M.CHOREV et al., pp. 8075-8076

## Description

This invention relates to new analogues of thymopentin (TP5) containing in the peptide chain one or two retro-inverse bonds, as well as to the process for the preparation of such new compounds and to their employment in pharmaceutical formulations.

More particularly, a first object of the present invention consists of the thymopentin analogues having one of the following general formulas 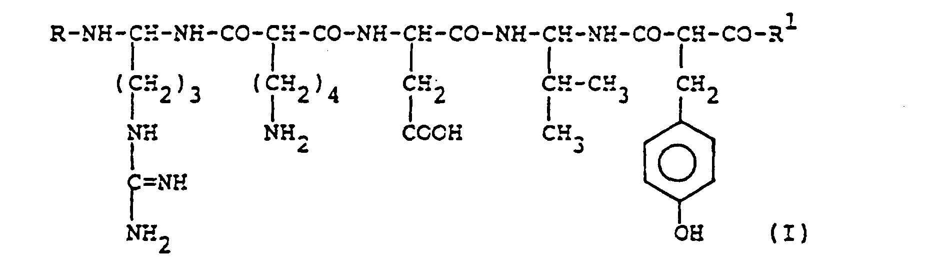 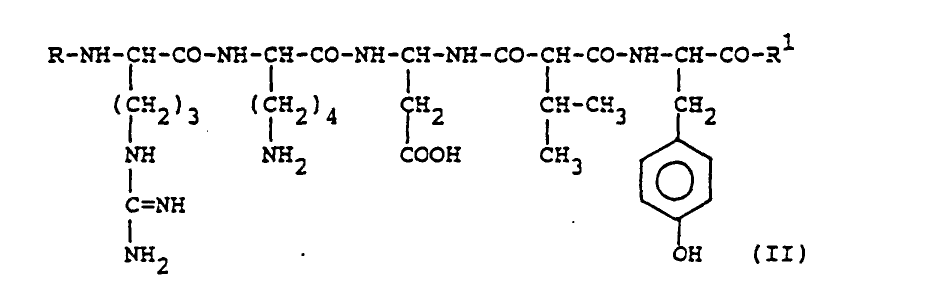 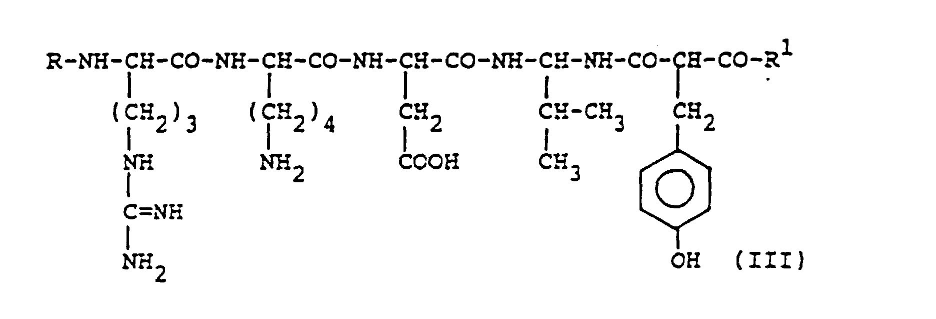 where R is hydrogen or an acyl radical, and
R¹ is a group -OR where R is a hydrogen atom or an alkyl group with a straight or a branched chain containing 1-6 carbon atoms, an alkenyl or an alkynyl radical having a straight or a branched chain containing from 3 to 6 carbon atoms, or an aryl-alkyl radical or an alkyl-aryl radical, which contain from 7 to 12 carbon atoms,
and the corresponding acid or basic addition salts pharmaceutically acceptable.

The new compounds of the present invention as defined above by the chemical structural formula that characterizes the same, can be pointed out in a more concise way through the employment of the internationally recognised set of symbols for the peptides, like:

R-H-gArg-mLys-Asp-gVal-mTyr-R¹ (I)

R-H-Arg-Lys-gAsp-mVal-Tyr-R¹ (II)

R-H-Arg-Lys-Asp-gVal-mTyr-R¹ (III)

where R and R¹ have the meanings specified above.

In such formulas, gArg points out a geminal diamino residue which can be derived from the amino acid arginine through substitution of the carboxyl group with an amino group, while mLys points out a malonyl residue substituted in the 2-position with the side chain of the amino acid lysine.

Similarly, gAsp and gVal point out a geminal diamino residue which can be derived from aspartic acid and valine, respectively, by substitution of the carboxyl group with an amino group, while mVal and mTyr point out a malonyl residue which is substituted in the 2-position with the side chain of the amino acids valine and tyrosine respectively.

As to the objects of the present invention, the term "acyl radical" identifies the acyl radicals deriving from alkanoic acids having a straight or a branched chain containing 1-6 carbon atoms, as for instance formyl, acetyl, propionyl, succinoyl, etc., and the aromatic acyl radicals deriving from benzoic acid and from substituted benzoic acid, as for instance benzoyl, 4-nitro-benzoyl, 2,3,4-trimethoxybenzoyl, and so on.

The term "pharmaceutically acceptable salts" as employed herein points out the acid or basic addition salts in which the anion or the cation respectively are relatively non toxic and innocuous when the compounds are administered just in the form of addition salts to the therapeutically active doses, so that any possible side-effects of the anions or the cations do not affect the advantageous effects of the active principle.

Among the various acids which are capable of forming pharmaceutically acceptable acid-addition salts with the compounds of the formulas (I), (II), and (III), the inorganic acids can be mentioned as examples, like hydrochloric acid, hydrobromic acid, phosphoric acid, sulfuric acid, etc., as well as the carboxyl organic acids like formic acid, acetic acid, propionic acid, lactic acid, malonic acid, succinic acid, etc., and the sulfonic organic acids like for instance methansulfonic acid or naphthalensulfonic acid.

The bases which are capable of forming pharmaceutically acceptable salts with the compounds of the formulas (I), (II), and (III), comprise for instance the inorganic bases like sodium or potassium hydroxides, ammonium hydroxide etc., and the organic bases like for instance triethylamine, triethanolamine, etc. Such addition salts can be obtained either directly through the process for the synthesis of the new retro-inverse peptides, or according to conventional methods starting from the compounds of the formulas (I), (II), and (III), in the form of the free bases or acids, by treatment with one or more equivalents of the acid or of the basis selected. If desired, it is also possible to convert a given acid addition salt into another one, by treatment of the first one with a suitable ion-exchange resin as disclosed for instance by R.A. Boissonas et al. in Helv.Chim.Acta, 43, 1349 (1960).

A preferred group of compounds of the present invention comprises the compounds of the formulas (I), (II) and (III), wherein R is a hydrogen atom or a metabolically labile acyl radical or an acyl radical whose bond with the amino group is rapidly broken in the first steps of the metabolic path of the product, and which does not give toxic effects or anyway effects which are contra-indicated in the therapy, at the concentration at which said compound of the formulas (I), (II) or (III) gives the desired pharmacological effect, while R¹ is as defined above, and R is hydrogen.

A group of compounds which are more preferred comprises the compounds of the formulas (I), (II) or (III) wherein R represents a hydrogen atom, R¹ is as defined above, and R is hydrogen.

The compounds of this invention have shown to possess a remarkable activity on the immune system.

During the last fifteen years, G. Goldstein and his group have studied in detail the biological action and the possible pharmacological employment of a polypeptide hormone secreted by the epithelial cells of thymus, i.e. thymopoietin (G. Goldstein, Nature, 247, 11 (1974); D.H. Schlesinger et al., Cell, 5, 361 (1975); T. Audhya et al. Biochemistry, 20, 6195 (1981)), whose primary sequence is made up of 49 amino acids.

Thymopoietin performs various regulating actions in the organism, affecting neuromuscular transmission (G. Goldstein, Lancet, 2, 119 (1968)), the differentiation of lymphocytes T and B (M.P. Scheid et al., J. Exp. Med., 147, 1727 (1978)) and the immune response (C.Y. Lau et al., J. Immunol., 125, 1634 (1980)).

Studies on structure-activity relationships have shown that for the biological activity the whole sequence of thymopoietin 49 amino acids is not necessary, as the pentapeptide

H-Arg-Lys-Asp-Val-Tyr-OH

(Thymopentin - TP5) corresponding to the amino acid sequence 32-36, possesses the whole biological activity of the natural hormone both in vitro and in vivo (G. Goldstein et al., Science, 204, 1309 (1979).

Thymopentin was already introduced in the clinical application both in the treatment of autoimmune origin diseases like for instance rheumatoid arthritis, and as a stimulating agent of the organism defence system for instance in the treatment of primary immune deficiencies caused by the absence or by incomplete development of thymus, with the consequent alteration of maturation of T lymphocytes, and of the acute or recurrent virus affections, or as an adjuvant in vaccinations.

However, the establishment of the therapeutic record is made critical by difficulties deriving from the determination of the efficient dose, because the pharmacological effect can change strongly with the method and duration of the administration (T.Audhya et al. Surv.Immunol.Res., 4^{th} Suppl.,1, 17 (1985) e T. Audhya et al., Int. J. Peptide Protein Res., 22, 568 (1983)). The limiting example is that reported by Bolla et al. in Int.J.clin.Pharm.Res., IV (6), 431 (1984), according to which the effect that stimulates the production of the antibodies on the part of TP5 administered through subcutaneous route is fully suppressed if the same dose is administered intravenously. A possible cause of that is the short half-life of TP5 in the plasma. Indeed, the TP5 is rapidly degraded in the plasma (t_{1/2} = 1.5 min) by the action of various proteases (T.P.Tischio et al., Int.J.Peptide Protein Res., 14, 479 (1979)). A very strong research activity has been started in the most recent years in order to obtain TP5 analogues having an increased resistance to proteases. For instance, see the European patent application EP-A-135722 and the US patent 4505853 which relate to thymopentin analogues which are obtained by opportunely changing the single amino acids inside the peptide sequence.

Again to reach such object, a thymopentin analogue has been synthesized which contains a retro-inversion at the most labile bond in the peptide chain, i.e. the bond between the residue of arginine and that of lysine (cf. J.P.Tischio et al., Int.J.Peptide Protein Res. 14, pp 479-484 (1979) and in particular Figure 4). The compound so obtained, which keeps unaltered or even improved the immunomodulating activity of thymopentin, has shown much more stable with respect of peptidases then the corresponding non retro-inverse compound. In particular, for example, while the half-life of thymopentin in heparinized human plasma has turned out to be of 1.5 minutes, that of the analogue compound retro-inverted at the Arg-Lys bond under the same experimental conditions has shown to be of 22 minutes.

Though this is a remarkable improvement, it is also evident that the availability of other compounds having the pharmacological possibilities of thymopentin but endowed with half-lives longer than that of thymopentin, would allow the therapeutic employment of thymopentin to be rationalized at the utmost degree.

Now it has been surprisingly found that by inverting also the bond between valine and tyrosine in addition to the bond between arginine and lysine, like in compounds of formula (I), a product is obtained which, though keeps the immunostimulating activity spectrum of thymopentin, also is practically refractory to enzymic demolition. Moreover, it has also been found that by inverting the bond between aspartic acid and valine, like in the structural formula (II), or between valine and tyrosine, like in the structural formula (III), instead of the bond between arginine and lysine, analogue compounds of thymopentin are obtained, which differ from the latter by a longer half-life. In particular, for instance, the thymopentin analogue in which just the two Arg-Lys and Val-Tyr bonds have been retro-inverted (the compound of the example 1), when tested for stability to enzymic hydrolysis in heparinized human plasma in a test parallel to a test for thymopentin, has shown a half-life longer than 6 hours. In the same test, the analogues of thymopentin, retro-inverted at the Asp-Val bond (Example 2) and at the Val-Tyr bond (Example 3) have shown a half-life of about 7 and about 2 times as much as that of thymopentin respectively.

The compounds of the present invention are easily prepared from the corresponding compounds of the formula (Ia), (IIa), and (IIIa) 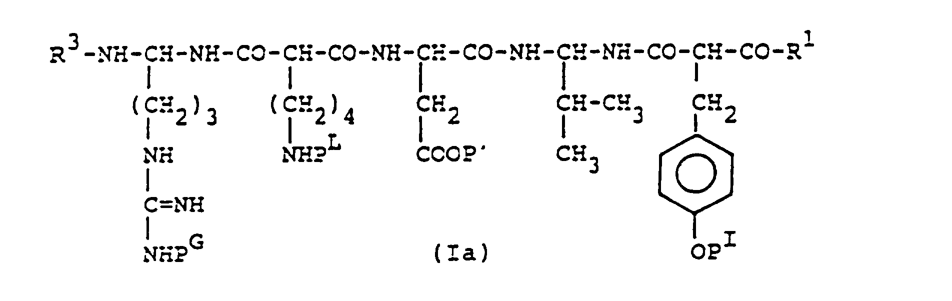 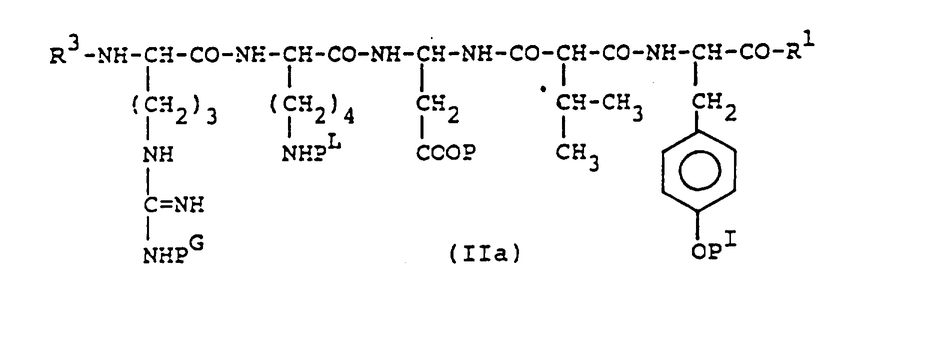 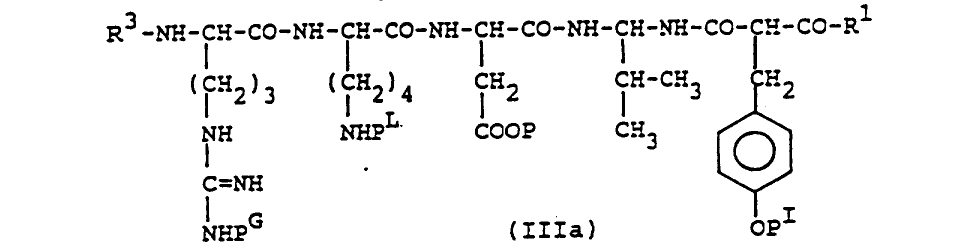 wherein:
R³ is R or a protection group of the alpha-amino function,
P^{G} has the meaning of a suitable protective group of the guanidine function,
P^{L} nas the meaning of protective group of the amino function in the side chain,
P is a protective group of the carboxyl function,
P^{I} is a protective group of the hydroxyl function of tyrosine, and
R¹ is as defined above,
   in order to remove the protective groups.

The removal of such protective groups is carried out according to methods that are well known in the field. In general, when conventional protective groups are employed, such as tert-butyl or tert-amyl for the carboxyl group, and for the hydroxyl group of tyrosine, the tert-butoxy carbonyl or benzyloxycarbonyl groups for the amino group of lysine, and the benzensulfonyl groups for the guanidino group of arginine, they are conveniently removed by acidolysis in an acid medium as for instance with hydrochloric acid diluted in acetic acid, with trifluoroacetic acid or with mixtures of trifluoroacetic acid and trifluoromethansulfonic acid, in the presence of small percentages of ethandithiole, anisole, thioanisole, or resorcinol, which are employed as "scavengers" for trapping the carbocations that are formed.

At the end of the reaction, the desired product corresponding to the formulas (I), (II) or (III), as it is or in the form of an addition salt, is recovered and then purified according to standard methods.

The homogeneity of the compounds so obtained is tested by tlc and by HPLC, and their purity is determined through the amino acid analysis and NMR.

The starting compound of the formula (Ia) wherein R³ is R, can be obtained by treatment with I, I-bis-trifluoroacetoxy-iodobenzene (TIB) of the corresponding terminal amide of the formula (IV) 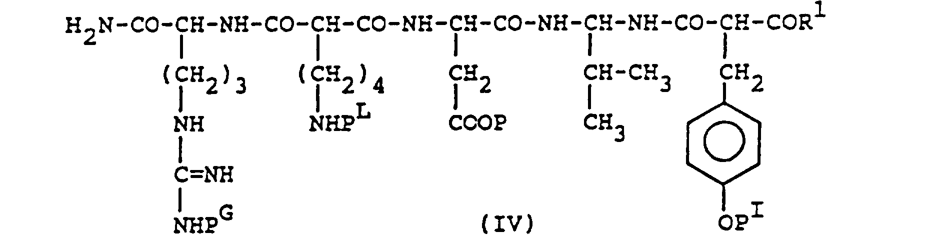 said treatment being followed possibly by acylation of the end amino function so formed. The intermediate of the formula (IV) can be prepared in its turn by condensation of a portion corresponding to the formula (V) 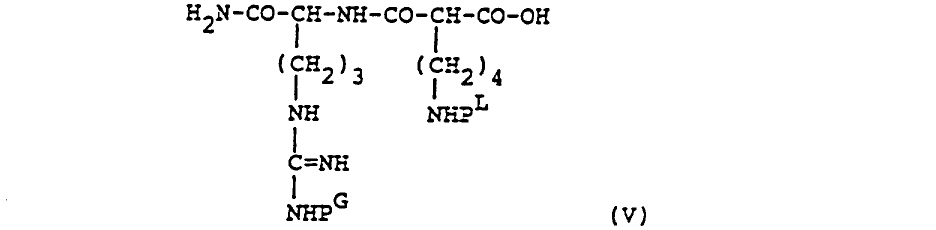 with a portion corresponding to the formula (VI) 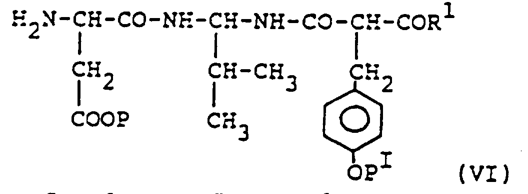 wherein P^{G}, P^{L}, P, P^{I} and R¹ are as defined above. Such condensation can be carried out in a suitable way according to any one of the procedures known in the technical literature for the peptide synthesis. Best results as regards yields and purities of the products obtained have been anyway reached through the employment of a carbodiimide like dicyclohexyl carbodiimide or diisopropylcarbodiimide and 1-hydroxybenzotriazole. In particular, the reaction is carried out by adding a slight excess amount of 1-hydroxybenzotriazole to a solution of the acid of the formula (V) kept at a low temperature, followed by the addition of said dicyclohexyl- or diisopropyl-carbodiimide, and then of the reaction partner of the formula (VI).

For such condensation reaction, which can be conveniently performed at room temperature, the standard aprotic polar organic solvents can be employed that are capable of dissolving the reactants and that do no interfere negatively with the behaviour of the reaction. Solvents of choice are dimethylformamide, acetonitrile, dimethylsulfoxide, possibly mixed with less polar solvents like for instance halogenated aliphatic hydrocarbons, e.g. methylene chloride, dichloroethane, etc.

Protective groups which can be conveniently employed in this synthesis are those conventionally employed, which are well known in the technical literature and are commonly employed in the classic peptide syntheses. In particular, P^{L} is preferably a tert-butoxycarbonyl group or a benzyloxycarbonyl group, which are possibly nitro- or halogen- substituted; P^{G} is preferably a benzene-sulfonyl group substituted in various ways as for instance an alkylbenzene sulfonyl group, e.g. a toluenesulfonyl group, or an alkylalkoxybenzene sulfonyl group, e.g. a 4-methoxy-2,3,6-trimethylbenzene sulfonyl group; P^{I} will preferably be a tert-butyl group, or a tert-amyl group, as such groups have shown to be stable against the action of TIB; and finally P can be any group capable of protecting the end carboxyl function as for instance an alkyl group, e.g. the tert-butyl or the tert-amyl group, or an aryl-alkyl group, for instance a benzyl group or a substituted benzyl group. When the condensation reaction, whose behaviour can be easily followed by tlc is complete, the product so obtained corresponding to the formula (IV) is recovered by means of standard procedures.

In particular, when according to a preferred aspect a carbodiimide is employed as the "coupling agent", such procedures provide the separation by filtering of the urea formed, as well as the evaporation of the solvent, the washing of the residue or of a solution of the same in a suitable organic solvent, with weakly alkaline or with weakly acid solutions, such operations being followed finally by the purification of the product through crystallization or chromatography.

The reaction of the product so obtained with TIB is then carried out according to the procedures disclosed in the Italian patent application 25755 A/81, which provide the reaction of the amide substrate with a slight excess amount of TIB in water mixtures of inert organic solvents as for instance dimethylformamide, acetonitrile and so on. The reaction is carried out by bubbling an inert gas, typically nitrogen, through the reaction mixture, and checking the behaviour of the reaction by tlc. When the complete conversion of the amide into the amine is observed, the organic solvent is removed, and the product can be easily recovered by lyophilization.

If a compound of the formula (I) is desired, in which R is an acyl group, the intermediate so obtained corresponding to the formula (Ia), wherein R³ is hydrogen, is then acylated by employment of the active esters of the acid R-OH, as for instance p-nitro-phenylester, 2,4,5-trichloro-phenylester, etc.

The compounds of the formulas (IIa) and (IIIa) can be prepared on the contrary starting from the corresponding portions or fragments of the formula (VII) 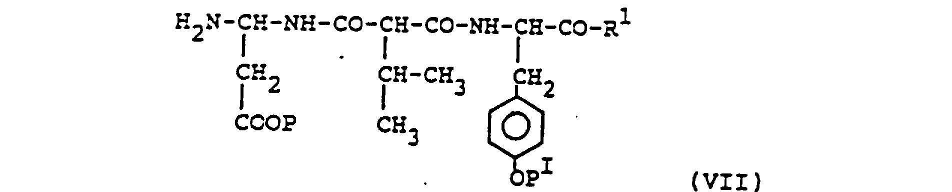 and (VI) 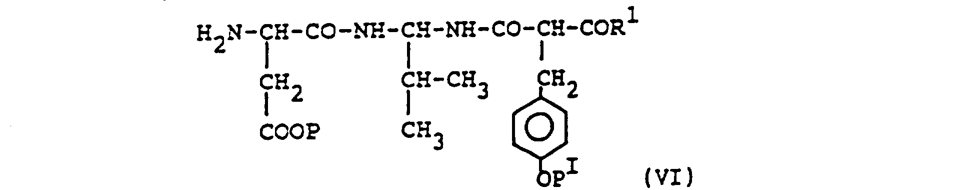 wherein P, P^{I}, and R¹ are as defined above, through successive condensations, according to classic procedures that are well known in the field of the peptide syntheses, with a lysine residue and an arginine residue which are protected in a suitable way, so as to give origin to intermediates of the general formulas (VIII) and (IX) respectively 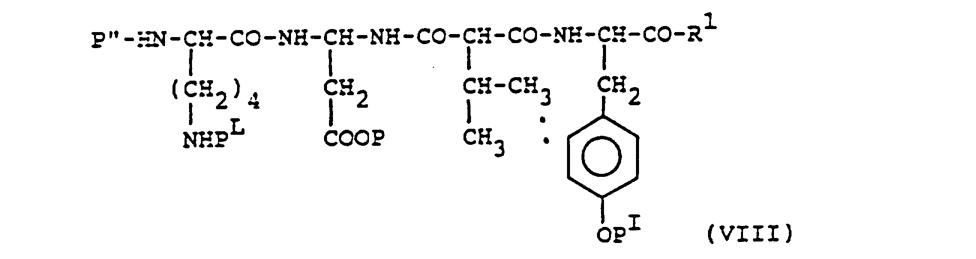 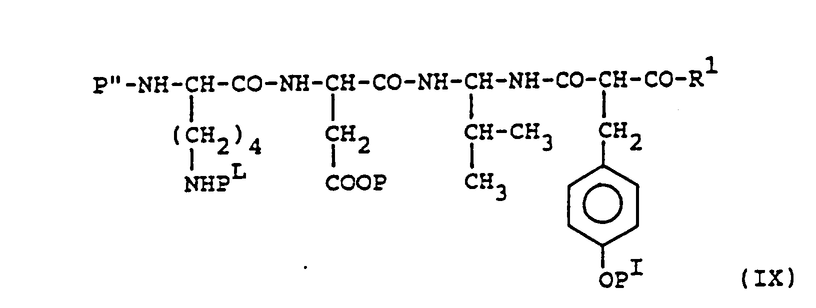 wherein P, P^{G}, P^{L}, P^{I} and R¹ have the meanings set forth above, and P" is hydrogen or a protective group of the alpha-amino function, which can be easily removed in conditions which do not give rise to the removal of the other protective groups. The starting compounds of the formulas (V) and (VI) in their terms can be easily synthesized both starting from compounds which are commercially available, and from compounds which are prepared purposely by means of procedures which are well known in the field of the organic and the peptide syntheses.

In particular, the fragment corresponding to the formula (V) can be conveniently prepared according to the disclosure given in the European Public. Number 282.891, while the fragments corresponding to the formula (VI) is prepared in a suitable way through condensation of an opportunely protected dipeptide corresponding to the formula (X) 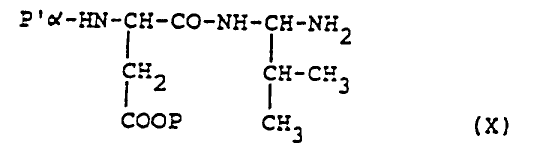 wherein P is a protective group of the carboxyl function, and P' is a protective group of the amino function in the alpha-position, with a compound of the formula (XI) 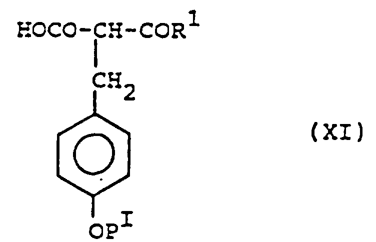 wherein R¹ and P^{I} are as defined above, such operation being followed by the removal of the protection of the primary amino function of the residue of the aspartic acid. This last compound can be prepared according to procedures that are well known in the technical literature for alkylating a malonic ester and for successively performing the hemisaponification of the same, or for the hemialcoholysis of a Meldrum acid opportunely substituted in the 5-position corresponding to the formula (XII) 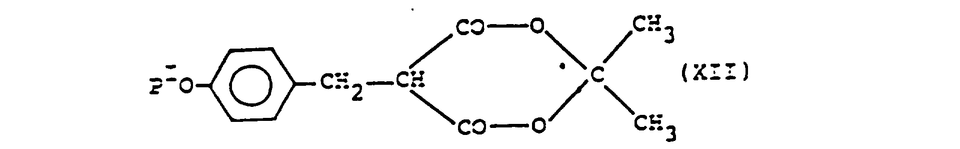 The intermediate of the formula (X) is prepared in its turn through condensation of an aspartic acid residue which is opportunely protected at the alpha-amino function and at the carboxyl function in the side chain, with valinamide, such operation being followed by the conversion of the intermediate amide into the primary amine by treatment with TIB.

The fragment of the formula (VIII) is prepared on the contrary by condensation of an amide of the formula (XIII) 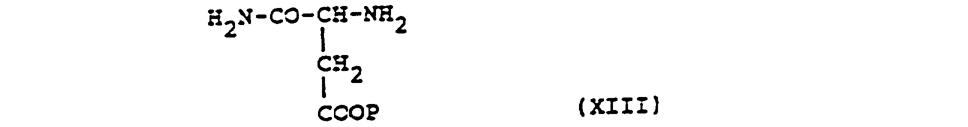 with a compound of the formula (XIV) 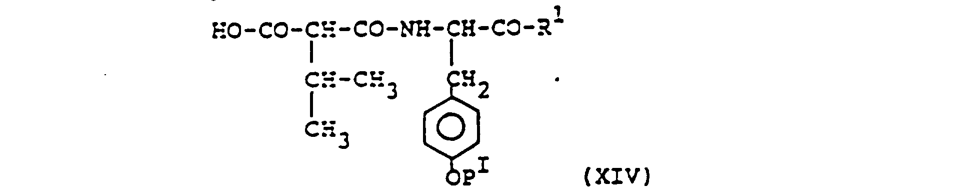 followed by the conversion of the amide group into the primary amino group by treatment with TIB. The compound of the formula (XIV) can be prepared in its turn by condensation of Meldrum acid opportunely substituted in the 5- position with the side chain of the amino acid valine, of the formula (XV) 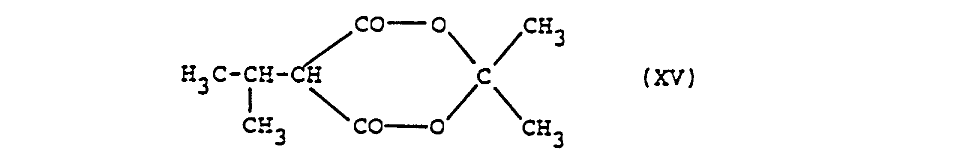 with a tyrosine opportunely protected corresponding to the formula (XVI) 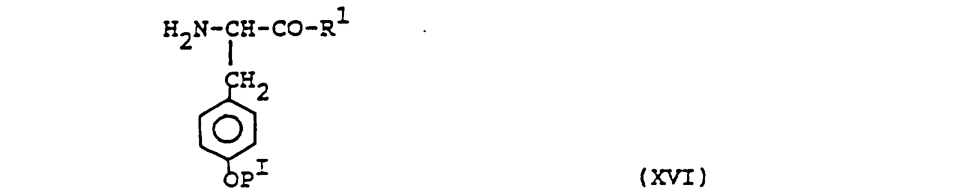 in the presence of a silanizing agent.

Also in the preparation of the compounds of the formulas (II) and (III), if a compound is desired wherein R is an acyl group, this can be easily obtained by removing the protection of the corresponding intermediate (IIa) and (IIIa) wherein R³ is an acyl group as defined above for R, which is obtained in its turn by acylation of the corresponding intermediate compound (IIa) and (IIIa), wherein R³ is hydrogen.

Accordingly, further objects of the present invention consist in a process for synthesizing the new compounds, as well as in the new intermediate compounds of the formulas (Ia), (IIa), (IIIa), (IV), (VI), (VII), (VIII), (IX), (X) and (XIV), which are obtained in such a process.

The compounds of the present invention can exist in several isomeric forms.

In the structural formulas (I), (II) and (III), there are indeed at least five asymmetric carbon atoms, but the absolute configuration of the other amino acids which are not involved in the retro-invertion, is the L-configuration (and such previously established configuration is simply obtained by employing in the synthesis the suitable L-amino acid derivatives), the absolute configuration of the gem-diamino carbon atoms is also established because the gem-diamino fragments are obtained starting from the corresponding D-amino acid amides, whereas the carbon atoms of the malonyl residues can have the R or the S configurations. Accordingly, the compounds of the present invention are obtained as mixtures of diastereoisomers (in particular, four for the compounds of the formula (I), and each two for the compounds of the formulas (II) and (III)), mixtures that can be anyway resolved, if desired, into the single isomers, according to the well known resolution methods.

The compounds of the formulas (I), (II) and (III) can be employed anyway both as the optically active form, and as the form of the mixture of isomers.

The compounds of the present invention showed to be more stable than thymopentin to the action of plasmatic peptidases.

In particular, the lability to enzymatic hydrolysis of

[gArg¹,(R,S)mLys,gVal⁴,(R,S)mTyr⁵]TP5,

[gAsp³,(R,S)mVal⁴]TP5 and [gVal⁴,(R,S)mTyr⁵]TP5,

has been tested in a test parallel to that for TP5, employing heparinized human plasma and incubating separately peptides at the concentration of about 30 nmoles/ml of plasma. The incubations were carried out at 37°C, and the drawings of the plasmatic mixtures, each one of 100 µl, were stopped at the various times by addition of trifluoroacetic acid in the ratio of 10%, with successive centrifugation at 10,000 x g, for five minutes. A portion of the supernatant is subjected to chromatography under conditions suitable to put into evidence the change in concentration of the peptide tested at the various time. The half-life, i.e., the incubation time at 37°C required for degrading by 50% the peptide tested, was calculated from the enzymic kinetics so obtained, such half-life being reported in the following Table I.

**Table I**

| Stability to enzymic hydrolysis in human plasma | |
|---|---|
| TP5 | (t/2 in min.) 1.5 |
| [gArg¹, (R,S)mLys, gVal⁴, (R,S)mTyr⁵]TP5 | > 360 |
| [gAsp³, (R,S)mVal⁴]TP5 | 10 |
| [gVal⁴, (R,S)mTyr⁵]TP5 | 2.5 |

The higher stability of the retro-inverse analogues with respect to TP5 has also been put into evidence by employing isolated enzymes (leucinaminopeptidase and carboxypeptidase).

The immunostimulating activity of such new analogues has been tested in vivo, in comparison to that of said TP5.

In particular, the so-called "hemolysis plate test" has been employed (PFC) performed according to the procedure disclosed by Jerne and Nordin.

Said test is carried out by administering through intravenous route to inbred C3H/HeNCrlBr male mice (Calco-Italia) aged 10-12 weeks and of body weight about 25 g, a pyrogen-free saline solution (0.2 ml) containing 1-2 x 10⁸ blood red cells of ram (SRBC). After two hours, groups of three mice each are treated p.o. (intragastric intubation) with 0.2 ml of saline solution (control) or with 0.2 ml of saline solution containing 100 ng of the peptide under test. After four days, the mice are sacrificed and the spleens are drawn and mechanically disrupted to separate the lymphocytes. The lymphocytes so isolated are washed with minimal soil containing Earle salts (MEM) (3 x 15 ml) and then resuspended into the same soil (1 ml) at a final concentration of 150,000 cells. Each cell suspension (0.1 ml) is then diluted 1/100 with MEM soil, and 100 µl of each dilution is added in double to wells of microplates containing 25 µl of MEM soil, 25 µl of a 10 % solution of SRBC antigens and 25 µl of guinea-pig complement at a final dilution of 1/64. The whole suspension is immediately transferred by capillarity from each well onto overlapped glass holder-slides. Such slides, with their edges sealed by means of paraffin, are then put for incubation into a thermostatic chamber at 37°C for one hour.

At the end of such time, the direct hemolysis plates are counted by means of a light-contrast viewer, such plates pointing out the number of lymphocytes which secrete antibodies (PFC). The antibodies so put into evidence are of the class of the IgM's, which are proper of the primary antibody response. The results, expressed as % with respect to the control sample, are reported in the following Table II.

**Table II**

| Compound | Plate formation % control |
|---|---|
| TP5 | 137 |
| (1,2-4,5)r.i.-TP5 | 196 |
| (3,4)r.i.-TP5 | 132 |
| (4,5)r.i.-TP5 | 190 |

In Table II, as well as throughout the text of the present invention, "r.i." points out the qualification "retro-inverse";
"(3,4)r.i.-TP5", accordingly, points out the analogue of the TP5, retro-inverted at the peptide bond 3-4, and
"(1,2-4,5)r.i.-TP5" points out the thymopentin analogue retro-inverted both at 1-2 bond and at the 4-5 bond.

Due to such biological features, the peptides of the present invention are to interfere with the body immune response, so as to stimulate substantially the same in case it is deficient. Accordingly, the compounds of the present invention are therapeutically useful in the treatment of a group of pathological states which are linked to an immune deficit. For instance, among such states there are the DiGeorge syndrome, characterized by the congenital absence of thymus, or the viral infections, the fungus-type or mycoplasmatic infections, as chronic or long-duration infections.

Accordingly, a further object of the present invention consists in the pharmaceutical compositions containing a therapeutically efficient amount of one or more compounds corresponding to the formulas (I), (II) and (III).

For the therapeutic use as immunostimulating or immunomodulating agents, the compounds of the present invention can be conveniently administered through the parenteral, the oral, or the sub-lingual routes.

The formulations containing the new compounds can be prepared according to the standard procedures, by combination of the active principle with an inert vehicle and possibly with some standard additives selected in a suitable way.

For oral or sub-lingual use, the compounds of the present invention can be administered in the form of tablets, capsules, drops, elixir, etc., prepared employing the conventional carriers/excipients like starch, sugars, water, alcohol etc., and possibly containing flavouring agents, stabilizers, preservatives, lubricants and so on. For parenteral use, the carrier of choice is sterile water for injections. Additives can also be added according to what is well known in the art. The daily dose therapeutically efficient will change according to the subject to be treated (weight, age, and condition) as well as according to the administration route. Anyway in general the compounds of the present invention are active when they are administered at a daily dose rate between 2 and 200 ng/ kg. Accordingly, the pharmaceutical formulations of the present invention will contain the compounds of the formulas (I), (II) or (III) in amounts suitable to ensure a correct daily dose rate inside the range specified above.

The following examples disclose in detail some compounds which are representative of the present invention as well as the method for the synthesis of the same.

### Example 1

### [gArg¹, (RS)mLys, gVal⁴, (R,S)mTyr⁵]TP5

### 1) Z-Asp(OBu^{t})-Val-NH₂

To a solution of Z-Asp(OBu^{t})OH (6.83 g, 20 mmoles) in tetrahydrofuran (THF; 40 ml) containing N-methyl-morpholine (NMM; 2.2 ml; 20 mmoles) cooled to -18°C and kept under nitrogen blanket, are added isobutyl-chloroformate (iBCF) at small portions (2.76 ml, 21 mmoles), keeping the temperature below -15°C.

Then a solution of H-Val- NH₂. HCl (3.36 g, 22 mmoles) in 10 ml THF containing NNM (2.4 ml, 22 mmoles) is added, and the temperature is allowed to rise up to the room temperature value. The reaction mixture is then treated with a water solution of NaHCO₃ at 5 % concentration (weight/volume) (50 ml) so obtaining a precipitate which is filtered and washed with the same solution (2 x 30 ml), with H₂O (3 x 30 ml), with 0.1 N HCl (3 x 30 ml) and again with H₂O (3 x 30 ml). The precipitate is then dried in an oven. 3.8 g of the product are obtained (yield 45 %) with melting point of 193-4°C, whose structure is confirmed by ¹H-NMR analysis.

The chromatographic analysis does not put into evidence any traces of impurities.

Tlc (chloroform/methanol/acetic acid, 85/10/5) (CMA) Rf = 0.7.
HPLC - column: Hibar, Lichrosorb® RP-18 (10 µ)

Eluting agents: gradient from 0 to 40 % of the solution B in A in the first 20 minutes, from 40 to 80 % of B in A in the next 10 minutes and then constant at the value of 80 % of B for further five minutes (B = 0.1 % TFA in CH₃CN; A . 0.1 % TFA in CH₃CN at 10 % in H₂O).
Flowrate: 1.5 ml/min.
Detection: U.V. at 230 nm
   a single peak with T_{R} = 22.85'

### 2) Z-Asp(OBu^{t})-gVal-H.CF₃COOH

A solution of [bis(trifluoracetoxy)iodo]benzene (TIB) (3 g, 6,98 mmoles) in CH₃CN (15 ml) is added at small portions to a suspension of the compound obtained in the step 1) (2.94 g, 6.98 mmoles) in an H₂O/CH₃CN mixture 2/1 (45 ml) kept stirred, under a nitrogen blanket. After three hours at room temperature, the solvent is removed and the residue is crystallized from diethyl ether (Et₂O). A white crystalline product is obtained (3.11 g, yield 85 %) with melting point of 104-5°C. The ¹H-NMR analysis confirms the assigned structure; while the HPLC analysis in the condition of the preceding step puts into evidence a single peak with T_{R} = 20.12'.

Tlc (CMA) Rf = 0.5.

### 3) HO-mTyr(Bu^{t})OBu^{t}

t-butyl alcohol (tBuOH) (566 µl, 6 mmoles), is added to a solution of 2,2-dimethyl-5-(para-tert-butoxy)benzyl-1,3-dioxane-4,6-dione [(M)Tyr(Bu^{t})] (918 mg, 3 mmoles) (prepared according to the procedure disclosed in the co-pending Italian patent application 23098 A/88) in toluene (2.4 ml). The reaction mixture is heated to 70°C and kept at this temperature for 18 hours. Then the mixture is taken to dryness and the oily residue so obtained is taken with ethyl acetate (AcOEt) (30 ml). The organic phase is washed with a water solution of NaHCO₃ at the concentration of 5 % (3 x 20 ml), then with a solution of citric acid at pH 4.5 (2 x 20 ml), and finally till neutrality with H₂O (2 x 20 ml). The mixture is dried over MgSO₄ and then the solvent is removed, so obtaining an oily product of a slight yellow colour (630 mg, yield 65 %), whose structure has been confirmed by mass-spectrometry and ¹H-NMR.

The HPLC analysis in the conditions of the step 1) puts into evidence a single peak with T_{R} = 27.79' (detection by U.V. at 254 nm).

Tlc (CMA) Rf = 0.59.

### 4) Z-Asp(OBu^{t})-gVal-(R,S)mTyr(Bu^{t})OBu^{t}

A solution of 1-hydroxybenzotriazole (HOBt) (301 mg, 2.1 mmoles) in N,N-dimethylformamide (DMF) (2 ml) and a solution of N,N'-dicyclohexylcarbodiimide (DCC) (397 mg, 1.925 mmoles) in DMF (3 ml) are added to a solution of the compound of the preceding step (620 mg, 1.92 mmoles) in DMF (5 ml) cooled to 0°C and kept stirred under nitrogen blanket. After 60 minutes the ice bath is removed, and the stirring is continued for another 60 minutes. Then a solution of the compound obtained in step 2) (888 mg, 1.75 mmoles) in DMF (5 ml) containing triethylamine (TEA) (246 µl, 1.75 mmoles) is added.

After 20 hours of stirring at room temperature, the N,N'-dicyclohexylurea (DCU) formed is filtered off and the filtrate is taken to dryness. The residue so obtained is taken with 70 ml of AcOEt and then treated with a water solution of 5 % NaHCO₃ (50 ml) for 20 minutes at room temperature, then the organic phase is separated, then washed with the same solution (2 x 50 ml), then with a water solution saturated with NaCl (3 x 50 ml), with a solution of 0.1 N HCl (3 x 50 ml) and finally again with a water solution saturated with NaCl (3 x 50 ml).

The organic phase is then dried over MgSO₄ and then taken to dryness, so leaving behind a slightly yellow solid (690 mg, yield 51 %).

The mass spectrometry and the ¹H-NMR analysis confirmed the assigned structure.

The HPLC analysis in the conditions of the step 1) puts into evidence a double peak caused by the pair of diastereoisomers with T_{R} = 32.35' and 32.53'.

Tlc (CMA) Rf = 0.37.

### 5) H-Asp(OBu^{t})-gVal-(R,S)mTyr(Bu^{t})OBu^{t}

To a solution of the compound obtained in the preceding step (690 mg, 0.89 mmoles) in a CH₃OH/CH₃CN mixture 1/1 (50 ml), a solution of ammonium formate is added (112 mg, 1.78 mmoles) in CH₃OH/H₂O 6/1 (3.5 ml), and, at small portions, palladium-on-active charcoal (Pd/C) at 10 % by weight (350 mg). The reaction mixture is then kept at room temperature and stirred under nitrogen blanket for 30 minutes, and then it is filtered over celite. The filtrate is then taken to dryness and the residue so obtained is taken with H₂O (70 ml) and taken to pH 9 by addition of a water solution of Na₂CO₃ at 5 % concentration. The water phase is then extracted with AcOEt (3 x 70 ml) and the organic phases collected are then dried over MgSO₄ and taken to dryness. 500 mg of the desired product are obtained, in quantitative yield.

The chromatographic analysis does not put into evidence any traces of impurities.

The analysis by HPLC in the conditions of the step 1) puts into evidence a single peak with T_{R} = 27.59'.

Tlc (CMA) Rf = 0.31.

### 6) Fmoc-D-Arg(Mtr)-NH₂

A solution of the ammonium salt of 1-hydroxybenzotriazole (BOBt.NH₃) (3.0 g, 16.45 mmoles) in 10 ml of DMF and a solution of DCC (3.38 g, 16.45 mmoles) in DMF (10 ml) are added to a solution of Fmoc-D-Arg(Mtr)-OH (10 g, 16.45 mmoles) in DMF (50 ml) cooled to 0°C and kept at that temperature under stirring. After about one hour, the temperature is allowed to rise up to the value of room temperature and the stirring is continued for an additional hour.

Then, the DCU so formed is removed by filtering off the same, and the solvent is evaporated off, so obtaining an oily residue which is taken with 70 ml AcOEt and then washed first with a water solution of NaHCO₃ at 5 % concentration (3 x 50 ml) and then with a water solution saturated with NaCl (3 x 50 ml). The organic solution is then dried over MgSO₄ and taken to dryness. The solid residue that is so obtained is triturated with Et₂O (100 ml) so giving a colourless powder (9.2 g, yield 93 %).
Melting point 168-172°C.

The HPLC analysis in the conditions of step 1) puts into evidence a single peak with T_{R} = 26,19'.

Tlc (CMA) Rf = 0.44.

### 7) H-D-Arg(Mtr)-NH₂.HCl

A suspension of the compound of the preceding step (9 g, 15.5 mmoles) in a DMF/diethylamine mixture 80/20 (100 ml) is kept under stirring for one hour. Then the solvent is removed by evaporation under reduced pressure and the residue is taken with AcOEt (100 ml). The organic solution is extracted with a water solution of 0.1 N HCl (3 x 50 ml) and the combined water extracts are washed with AcOEt (50 ml) and lyophilized a number of times so as to obtain a flaky product (4.5 g, yield 80 %).

The product has no exact melting point.

The ¹H-NMR analysis confirms the assigned structure. The HPLC analysis in the conditions of the step 1) puts into evidence a single peak with T_{R} = 12.69'.

Tlc (butanol/H₂O/acetic acid, 4/1/1- (BWA)) Rf = 0.36.

### 8) HO-(R,S)mLys(Boc)-OEt

The compound corresponding to the title is prepared starting from N-tert-butoxy-carbonyl-4-chloro-butylamine and form diethylmalonate, following substantially the procedure disclosed in EP-A-253,190, Example 1, step c).

The HPLC analysis in the conditions of step 1) puts into evidence a single peak with T_{R} = 19.58'.

Tlc (CMA) Rf = 0.58.

### 9) H₂N-D-Arg(Mtr)-(R,S)mLys(Boc)-OEt

A solution of HOBt (0.76 g, 6.3 mmoles) in DMF (10 ml) and a solution of DCC (1 g, 4,85 mmoles) in 5 ml of DMF, are added to a solution of the compound of the preceding step (1.47 g, 4.85 mmoles) in 15 ml of DMF cooled to 0°C and kept stirred under nitrogen blanket. After one hour, the temperature is allowed o rise up to the value of room temperature, and the stirring is continued for an additional hour. Then a solution of the compound obtained in step 7) (1.86 g, 4.4 mmoles) in DMF (15 ml) containing TEA (0.612 ml, 4.4 mmoles) is added. The reaction mixture is left under stirring at room temperature for 20 hours, then an excess amount of DCC is added (0.2 g, 0.97 mmoles) and the stirring is continued for an additional period of 4 hours. Then the DCU so formed is filtered off, the solvent is evaporated at reduced pressure and the residue is taken with THF (20 ml). The mixture is cooled down to -15°C and left at that temperature overnight, then it is filtered again and the THF is evaporated off the filtrate. The oily residue so obtained is taken with AcOEt (70 ml) and the organic solution is washed sequentially with a 5% NaHCO₃ solution (3 x 50 ml), with a water solution saturated with Nacl (3 x 50 ml), a water solution of 0.1 N HCl (3 x 50 ml) and finally again with the solution saturated with NaCl (3 x 50 ml). The organic phase washed is then dried over MgSO₄ and then evaporated to dryness. The oily residue is then triturated with hexane, so giving a white, finely divided powder (2.26 g, yield 76%).

Melting point 148-9°C.

The ¹H-NMR and the mass-spectrometry analyses confirm the assigned structure.

The HPLC analysis in the conditions of step 1) puts into evidence a single peak with T_{R} = 23.38'.

Tlc (CMA) Rf = 0.4

### 10) H₂N-D+Arg(Mtr)-(R,S)mLys(Boc)-OH

A solution of KOH (0.204 g, 3.63 mmoles) in absolute ethyl alcohol (EtOH) (5 ml) is added dropwise during 90 minutes to a solution of the compound of the step 9) (2.21 g, 3.3 mmoles) in 20 ml of absolute EtOH cooled to 0°C and kept under nitrogen.

After 16 hours, the reaction mixture is diluted with 50 ml of H₂O, then taken to small volume and extracted with Et₂O (3 x 50 ml). The water phase is acidified with 0.1 N HCl till pH 3 and then extracted again with AcOEt (3 x 50 ml). The organic extracts are collected together, dried over NgSO₄ and evaporated. An oily residue is obtained (1.97 g, yield 93 %) whose structure has been confirmed by ¹H-NMR and mass-spectrometry analyses. The HPLC analysis under the conditions of step 1) puts into evidence a double peak given by the pair of diastereoisomers with T_{R} = 20.02' and 20.52'.

Tlc (CMA) Rf = 0.13 and 0.19

### 11) H₂N-D-Arg(Mtr)-(R,S)mLys(Boc)-Asp(OBu^{t})-gVal-(R,S)mTyr(Bu^{t})OBu^{t}

A solution of HOBt (157 g, 1.1 mmoles) in DMF (2 ml) and a solution of DCC (206 g, 1 mmoles) in DMF (1.8 ml) are added to a solution of the compound obtained in the preceding step (643 mg, 1 mmole) in DMF (3.2 ml) previously cooled to 0°C and kept under a nitrogen blanket. After one hour, the temperature is allowed to rise up to the value of room temperature and the solution is kept stirred for an additional hour. Then, a solution of the compound obtained in step 5 (500 mg, 0.89 mmoles) in DMF (10 ml) is added, and after 20 hours of stirring, the DCU so formed is removed by filtering and the solvent is evaporated off. The residue so obtained is taken with THF (15 ml), then cooled down to - 15°C overnight and filtered again. After removing the THF, the residue is taken with AcOEt (150 ml) and then treated with a 5 % NaHCO₃ water solution (50 ml) for 20 minutes at room temperature; the organic phase id then separated and washed with the same solution (2 x 50 ml), then with a water solution saturated with NaCl (3 x 50 ml), with a 0.1 N HCl solution (3 x 50 ml) and finally with H₂O (3 x 50 ml).

The organic phase is then dried over MgSO₄ and taken to dryness, so leaving behind a residue which is triturated with Et₂O. Thus a powder is obtained which is off white in colour and has a melting point of 171-4°C (574 mg, yield 54 %).

The ¹H-MNR and mass-spectrometry analyses confirm the assigned structure.

The HPLC analysis under the conditions of the step 1) puts into evidence a single peak with T_{R} = 31.36'.

Tlc (CMA) Rf = 0.54.

### 12) TFA.H-gArg(Mtr)-(R,S)mLys(Boc)-Asp(OBu^{t})-gVal-(R,S)mTyr(Bu^{t})OBu^{t}

The compound obtained in the preceding step (574 mg, 0.48 mmoles) is dissolved into a mixture containing CH₃CN (3 ml), DMF (1 ml) and H₂O (2.5 ml) kept at room temperature and under a nitrogen blanket. Then a solution of TIB (245 mg, 0.57 mmoles) in CH₃CN (1 ml) is added. After three hours, a further portion of TIB is added, which is equal to 20 % (49 mg, 0.11 mmoles), and the solution is kept under stirring for two additional hours. The reaction mixture is then taken to dryness and the residue is taken with CH₃OH (20 ml) and taken again to dryness a number of times. The residue so obtained contains the desired compound, at a purity higher than 85 % (as determined by HPLC under the conditions of step 1), where a double peak is put into evidence, with T_{R} = 31.78' and 31.99'.

The product is employed as it is obtained without any further purification, in the successive deblocking step.

### 13) CH₃COOH.H-g-Arg-(R,S)mLys-Asp-gVal-(R,S)mTyr-OH

About 400 mg of the product obtained in the preceding step (about 0.25 mmoles) is treated for 20 minutes at room temperature and under nitrogen blanket with a freshly prepared mixture containing trifluoroacetic acid, trifluoromethan-sulfonic acid and 1,2-ethandithiole (TFA/TFMSA/EDT, 89/1/10) (20 ml). After 20 minutes, the reaction mixture is cooled down to 0°C, and TEA (0.3 ml, 1.44 mmoles) is added dropwise to the same, then the solution is taken to dryness under nitrogen flow. The residue is taken with H₂O (70 ml), the water solution is extracted with Et₂O (50 ml) and the ethyl ether phase is back washed with H₂O (30 ml). The two water phases are combined and washed with Et₂O (3 x 50 ml). Water is then evaporated off under reduced pressure and the oily residue is purified by ion exchange chromatography over a column (15 x 0.9 cm) packed with CM-Sephadex C-25 (2 g) and developed with a linear gradient in ammonium acetate at pH 5 from 0.1 to 0.6 M in 8 hours at a flowrate of 0.8 ml/min. The fractions containing the desired product are collected, concentrated down to a small volume and then lyophilized a number of times.

Thus 74 mg of the product is obtained (yield 40 %). The mass and ¹H-NMR analyses confirm the structure of the product, while the HPLC analysis under the standard conditions of step 1) confirm its purity, putting into evidence two peaks only which are given by the pairs of diastereoisomers with T_{R} = 8.65' and 9.44'.

### Example 2

### [gAsp³, (R,S)mVal⁴]TP5

### 1) 2,2-dimethyl-5-isopropyl-1,3-dioxan-4,6-dione [(M)Val]

A solution of 2,2-dimethyl-1,3-dioxan-4,6-dione (Meldrum's acid) (14.4 g, 100 mmoles) in acetone (50 ml), containing pyridine (0.5 ml, 5 mmoles) and glacial acetic acid (0.3 ml, 5 mmoles) is stirred at room temperature for 6 hours, and then it is taken to dryness. NaBH₄ (4.16 g, 110 mmoles) is added to the residue which is taken with CH₃OH (150 ml). After 15 minutes, the volume of CH₃OH is reduced to about 50 ml, then H₂O (50 ml) is added and the solution is taken to pH 3 by adding 3 N HCl. Thus a white precipitate is obtained which is recovered by filtration and is dried in an oven (11.6 g, yield 63 %).

Melting point 98-99°C

The ¹H-NMR confirms the structure assigned. The chromatographic analysis under the same conditions as those pointed out in step 1) of Example 1 has put into evidence a single peak with T_{R} = 18.27'.

Tlc (CMA) Rf = 0.34

### 2) Fmoc-D-Asp(Obu^{t})NH₂

HOBt.NH₃ (3.21 g, 20 mmoles) and a solution of DCC (4.13 g, 20 mmoles) in DMF are added to a solution of Fmoc-D-Asp(OBu^{t})OH (8.23 g, 20 mmoles) in DMF (60 ml), cooled to 0°C and kept stirred under nitrogen blanket. After about 60 minutes, the ice bath is removed and the solution is kept stirred for another period of 60 minutes at room temperature, then the DCU so formed is filtered off and the solvent is removed by evaporation under reduced pressure. The reaction raw product is taken with AcOEt (150 ml), extracted with a 5 % NaHCO₃ water solution (3 x 100 ml), and then with a solution saturated with NaCl (3 x 50 ml). The organic phase is dried over MgSO₄, then taken to dryness so as to obtain a white gelatinous mass that is triturated with H₂O and dried in an oven (7.8 g, yield 95 %). Melting point = 134-136°C.

The ¹H-NMR analysis confirms the assigned structure. The chromatographic analysis under the same conditions as those of step 1) of Example 1, puts into evidence a single peak with T_{R} = 27.03'.

Tlc (CMA) Rf = 0.62

### 3) H-D-Asp(OBu^{t})NH₂

The product obtained in the preceding step (7.5 g, 18 mmoles) is treated for 60 minutes at room temperature and under nitrogen blanket with a DMF/diethylamine mixture 80/20 (100 ml). After one hour the reaction mixture is taken to dryness, the residue is taken with AcOEt (100 ml) and extracted with 0.1 N HCl (3 x 70 ml). The water phases combined and reduced to a volume of about 100 ml are adjusted to pH 9 with a 10 % Na₂CO₃ water solution and re-extracted with AcOEt (4 x 50 ml).

The organic phases combined, dried over MgSO₄ and evaporated under reduced pressure leave behind an oily residue which is purified by flash chromatography over silica, eluting with AcOEt and then with CH₃OH. Thus 1.45 g of a yellowish oil is obtained (yield 44 %).

The mass and ¹H-NMR analyses confirm the structure assigned.

The HPLC analysis in the conditions of step 1) of Example 1 puts into evidence a single peak with T_{R} = 6.52'

Tlc (BWA) Rf = 0.48

### 4) H-Tyr(Bu^{t})OBu^{t}

To a solution of Z-Tyr(Bu^{t})OBu^{t}, (prepared according to the method disclosed in "New Aspects in Physiological Antitumor Substances", Karger Basel (1985), p.33) (6.92 g, 16 mmoles) in CH₃OH (60 ml), a solution of ammonium formate (2.52 g, 40 mmoles) in CH₂OH (40 ml) and Pd/C 10 % (3.5 g) are added. The resulting suspension is kept under stirring at room temperature and under nitrogen blanket for 30 minutes, then it is filtered on celite and the solvent is removed by evaporation under reduced pressure. The oily residue so obtained is taken with a 10 % Na₂CO₃ water solution (50 ml) and extracted with AcOEt (4 x 50 ml); the organic phases are then combined, dried over MgSO₄ and the solvent is removed by evaporation under reduced pressure. Thus 3.6 g of a colourless oily product is obtained (yield 77 %).

The ¹H-NMR analysis confirms the structure assigned. The HPLC analysis under the same conditions as those of step 1) of Example 1 shows a single peak at T_{R} = 20.90'.

Tlc (CMA) Rf = 0.44

### 5) HO-(R,S)mVal-Tyr(Bu^{t})OBu^{t}

To a solution of H-Tyr(Bu^{t})OBu^{t} (1.467 g, 5 mmoles) in THF (25 ml), the addition is performed in succession of (M)val (1.012 g, 5.5 mmoles), N,O-bis-(tritmethylsilyl)acetamide (BSA) (2.45 ml, 10 mmoles) and trimethylsilyl chloride (TMSC) (0.634 ml, 5 mmoles).

The reaction is carried out under nitrogen blanket at room temperature for 20 hours, then H₂O is added (50 ml) and the pH value is made equal to 4 with citric acid. Then the solution is extracted with methylene chloride (3 x 50 ml), the organic phases are then collected, washed with water (50 ml), dried over MgSO₄, then evaporated under reduced pressure. 2.02 g of a colourless oil is obtained (yield 96 %).

The mass and ¹H-NMR analysis confirm the structure assigned.

The HPLC analysis under the same conditions of step 1) of Example 1 puts into evidence a single peak at T_{R} = 27.80'.

Tlc (CMA) Rf = 0.6

### 6) H₂N=D-Asp(OBu^{t})=(R,S)mVal-Tyr(Bu^{t})OBu^{t}

A solution of HOBt (0.567 g, 3.96 mmoles) in DMF (3 ml) and a solution of DCC (0.73 g, 3.53 mmoles) in CH₂Cl₂ (5 ml) are added to a solution of the product obtained in step 5) (1.49 g, 3.53 mmoles) in CH₂Cl₂ (20 ml), cooled to 0°C and kept stirred under nitrogen blanket. After 60 minutes the ice bath is removed and the solution is kept stirred for an additional period of 60 minutes; then a solution of H-D-Asp(OBu^{t})NH₂ (the D-aspartic acid amide whose carboxyl group in the said chain is protected in the form of the tert-butyl ester) (0.62 g, 3.3 mmoles) in CH₂Cl₂ (7 ml) is added and the reaction is carried out at room temperature for 20 hours.

The DCU formed is filtered off, the solvent is removed by evaporation under reduced pressure and the reaction raw product is taken with THF (30 ml); then the mixture is cooled down to -15°C for two hours, the DCU precipitate is filtered off, the solvent is removed by evaporation under reduced pressure and the residue is taken with AcOEt (75 ml). Then the solution is washed with a 5 % NaHCO₃ water solution (3 x 50 ml), with a solution saturated with NaCl (3 x 50 ml), with 0.1 N HCl (3 x 50 ml) and finally with H₂O (3 x 50 ml). The organic phase is then dried over MgSO₄, the solvent is evaporated off and the residue is triturated with n-hexane (50 ml). A white solid is obtained (1.28 g, yield 65 %) with melting point 113-5°C.

The mass and ¹H-NMR analyses confirm the structure assigned.

The HPLC analysis under the same conditions as those of step 1) of Example 1, puts into evidence a single peak at T_{R} = 29.21'.

Tlc (CMA) Rf = 0.62

### 7) TFA.H-gAsp(OBu^{t})-(R,S)mVal-Tyr(Bu^{t})OBu^{t}

A solution of TIB (0.807 g, 1.88 mmoles) in CH₃CN (5 ml) is added at small portions to a solution of the compound obtained in the preceding step (1.01 g, 1.706 mmoles) in a CH₃CN/H₂O mixture 2/1 (15 ml) which is kept stirred under nitrogen blanket. After 3.5 hours at room temperature, the solvent is removed and the residue is triturated with an ethyl ether/n-hexane mixture 1/1 (20 ml). A white product is thus obtained (0.815 g, yield 70 %) with melting point 143-45°C.

The mass and ¹H-NMR analyses confirm the structure assigned.

The HPLC analysis under the same conditions as those of step 1) of Example 1 puts into evidence a single peak at T_{R} = 28.03'.

Tlc (CMA) Rf = 0.48

### 8) Z-Lys(Boc)-gAsp(OBu^{t})-(R,S)mVal-Tyr(Bu^{t})OBu^{t}

A solution of HOBt (193 mg, 1.34 mmoles) in DMF (2 ml) and a solution of DCC (254 mg, 1.23 mmoles) in DMF (2 ml) are added to a solution of Z-Lys(Boc)-OH (470 mg, 1.23 mmoles) in DMF (4 ml) cooled to 0°C and kept stirred under nitrogen blanket.

After 30 minutes the temperature is allowed to rise up to the value of room temperature and the solution is kept stirred for an additional period of 30 minutes.

Then a solution of the compound obtained in the preceding step (770 mg, 1.12 mmoles) in DMF (5 ml) containing TEA (123 µl, 1.12 mmoles) is added. The reaction is carried out at room temperature for 20 hours, then the DCU formed is filtered off and the solvent is evaporated under reduced pressure, so that a residue is obtained which is then taken with THF (10 ml). The solution so obtained is cooled to -15°C overnight, then filtered again and taken again to dryness. The residue is taken with AcOEt (70 ml) and the organic solution so obtained is washed first with a 5 % sodium bicarbonate water solution (3 x 50 ml), then with a sol-ution saturated with sodium chloride (3 x 50 ml), then with a 0.1 N HCl water solution (3 x 50 ml), and finally again with the water solution saturated with NaCl (3 x 50 ml).

The organic phase is then dried over MgSO₄ and taken to dryness. The gelatin-like residue so obtained is triturated with Et₂O so as to give an off white solid (735 mg, yield 71 %) characterized by a melting point of 190-3°C.

The mass and ¹H-NMR analyses confirm the structure assigned.

The HPLC analysis under the same conditions as those of step 1) of Example 1, points out a single peak with T_{R} = 33.90'

Tlc (CMA) Rf = 0.73

### 9) HCOOH.H-Lys(Boc)-gAsp(OBu^{t})-(R,S)mVal-Tyr(Bu^{t})-OBu^{t}

To a solution of the compound obtained in the preceding step (700 mg, 0.76 mmoles) in a CH₃OH/DMF mixture 7/1 (21 ml), the addition is performed of ammonium formate (95 mg, 1.52 mmoles) dissolved in CH₃OH (3.5 ml) and H₂O (0.2 ml), and, at small portions, active charcoal-supported palladium at 10 % (350 mg). The reaction is carried out at room temperature and under nitrogen blanket. After 30 minutes the reaction mixture is filtered on celite and the solvent is removed by evaporation under reduced pressure. A white solid is thus obtained (518 mg, yield 91 %), whose HPLC analysis under the standard conditions of step 1) of Example 1 does not put into evidence any traces of impurities; single peak with T_{R} = 29.95'.

Tlc (CMA) Rf = 0.39

### 10) Boc-Arg(Mtr)-Lys(Boc)-gAsp(OBu^{t})-(R,S)mVal-Tyr-(Bu^{t})OBu^{t}

A solution of HOBt (120 mg, 0.84 mmoles) in DMF (3 ml) and a solution of DCC (159 mg, 0.77 mmoles) in DMF (2 ml) are added to a solution of Boc-Arg(Mtr)-OH (375 mg, 0.77 mmoles) in DMF (10 ml) cooled to 0°C and kept stirred under nitrogen blanket. After 30 minutes the temperature is allowed to rise to the value of room temperature and the solution is stirred for an additional period of 30 minutes. Then a solution of the compound obtained in step 9) (previously desalified by treatment with Na₂CO₃ at a pH of about 9 (50 ml) and extraction with AcOEt (3 x 30 ml)) (518 mg, 0.69 mmoles) in DMF (10 ml) is added.

The reaction is carried out at room temperature for 20 hours, then the DCU so formed is removed by filtration, the DMF is removed by evaporation under reduced pressure and the residue is treated as is disclosed in step 8). By evaporating the organic phase, about 490 mg of the product is obtained (yield 60 %) with melting point 179-182°C.

The HPLC analysis under the same conditions as those of step 1) of Example 1, points out a single peak at T_{R} = 34,10'.

Tlc (CMA) Rf = 0.77

The compound is employed as is obtained in the succissive deblocking step with no special purification procedures.

### 11) CH₃COOH.H-Arg-Lys-gAsp-(R,S)mVal-Tyr-OH

The product obtained from the preceding step (170 mg, 0.15 mmoles) is treated for 20 minutes at room temperature, under nitrogen blanket, with a freshly prepared solution containing trifluoroacetic acid (TFA), trifluoromethansulfonic acid (TFMSA) and 1,2-ethandithiole (EDT) (89/1/10) (20 ml). After 20 minutes the reaction mixture is cooled to 0°C and TEA (0.3 ml, 1.44 mmoles) is added dropwise to the same, then the mixture is taken to dryness under a nitrogen flow.

The residue is taken with H₂O (50 ml), extracted with Et₂O (30 ml) and the ethyl ether phase is back washed with H₂O (20 ml). The two water phases combined are extracted with Et₂O (3 x 30 ml) and then evaporated under reduced pressure. The oily residue so obtained is purified by ion exchange chromatography over a column (15 x 0.9 cm) packed with CM-Sephadex C-25 (2 g) and developed by means of a linear gradient in ammonium acetate at pH 4.4 from 0.15 to 0.6 M in 8 hours at a flowrate of 0.8 ml/min.

The fractions containing the desired product are combined, concentrated down to a small volume under reduced pressure and lyophilized a number of times. 60.5 mg of the product (yield 56 %) is obtained.

The ¹H-NMR and mass-spectrometry analyses confirm the structure of the product, while the HPLC analysis confirms the purity of the same under the standard conditions of step 1) of Example 1; a single peak with T_{R} = 8.21'.

### Example 3

### [gVal⁴,(R,S)mTyr⁵]TP5

The synthesis of this compound ((III): R = H, R = H) starting from the C-end tripeptide H-Asp(OBu^{t})-gVal-(R,S)mTyr(Bu^{t})OBu^{t} (obtained as disclosed in Example 1, steps 1)-5)) is completed in a suitable way by means of:
1) a condensation with a residue of the formula Z-Lys(Boc)-OH;
2) a catalytic hydrogenation for deblocking the protective group of alpha-NH₂;
3) a condensation with Boc-Arg(Mtr)-OH;
4) an end acid deblocking operation followed by purification through ion exchange chromatography.

The procedures employed in the steps 1)-4) disclosed above are fully similar to the procedure illustrated in the steps 8)-11) of Example 2.

Thus the desired product is obtained, whose structure has been confirmed by the mass and ¹H-NMR analyses.

The HPLC analysis under the standard conditions of step 1) of Example 1 points out two peaks which are given by the pair of diastereoisomers with T_{R} = 7.79' and 8.00'.

## Claims (Claims for the following Contracting State(s): AT, BE, CH, DE, DK, FR, GB, LI, LU, NL, SE)

1. A thymopentin analogue having one of the following formula 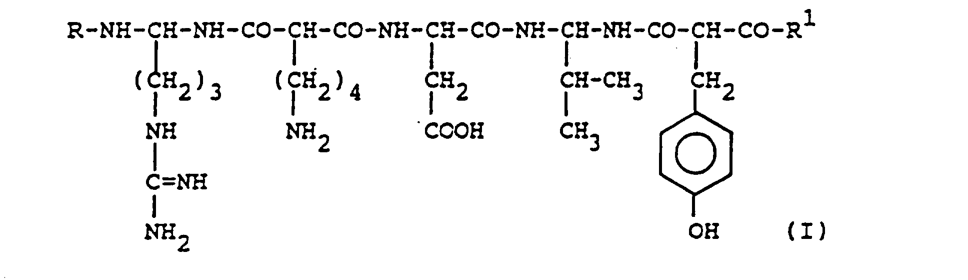 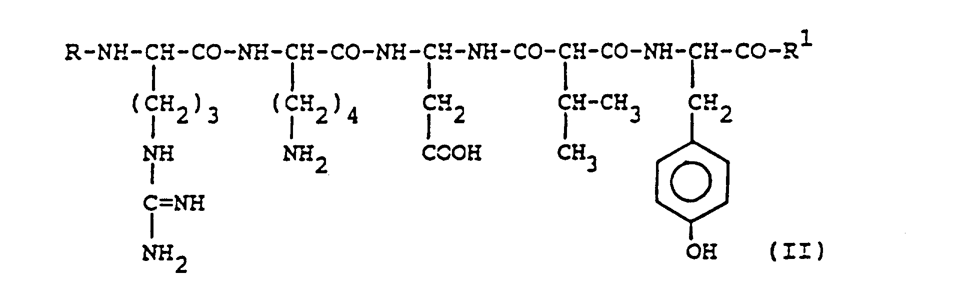 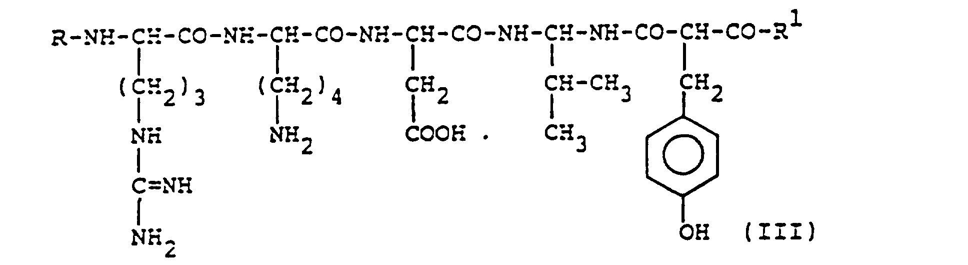 wherein R is hydrogen or a metabolically labile acyl radical or an acyl radical whose bond with the amino group is rapidly broken in the first steps of the metabolic path of the product, and
R¹ is a group of the formula -OR, wherein
R is a hydrogen atom or an alkyl radical having a straight or a branched chain containing from 1 to 6 carbon atoms, an alkenyl or an alkynyl radical having a straight or branched chain containing from 3 to 6 carbon atoms, or an aryl-alkyl or alkyl-aryl radical containing from 7 to 12 carbon atoms,
and the corresponding acid or basic addition salts pharmaceutically acceptable.

2. A compound according to claim 1 wherein R and R¹ are as defined in claim 1 and R is hydrogen.

3. A compound according to claim 2, wherein R is hydrogen.

4. A compound according to claim 3, which is selected from
[Arg¹, (R,S)mLys,gVal⁴,(R,S)mTyr⁵]TP5,
[gAsp³,(R,S)mVal⁴]TP5, [(R,S)mVal⁴,gTyr⁵]TP5,
and the addition salts thereof.

5. A compound according to claim 4, which is selected from
[gArg¹,(R,S)mLys ,gVal⁴,(R,S)mTyr⁵]TP5
and the addition salts thereof.

6. A pharmaceutical composition comprising a compound according to claim 1 as the active principle.

7. A compound according to claim 1 for employment as a drug.

8. A process for the preparation of a thymopentin analogue having one of the following general formulas 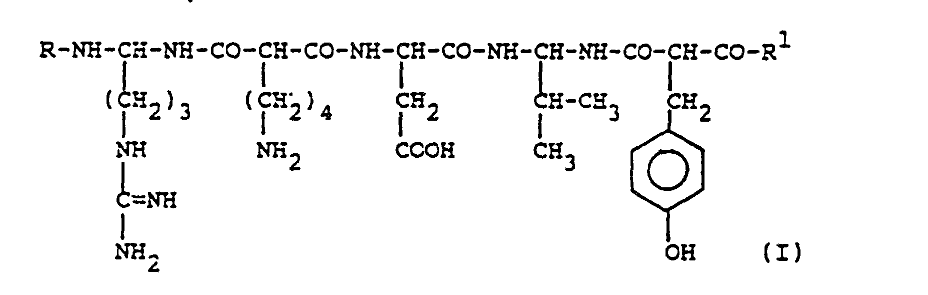 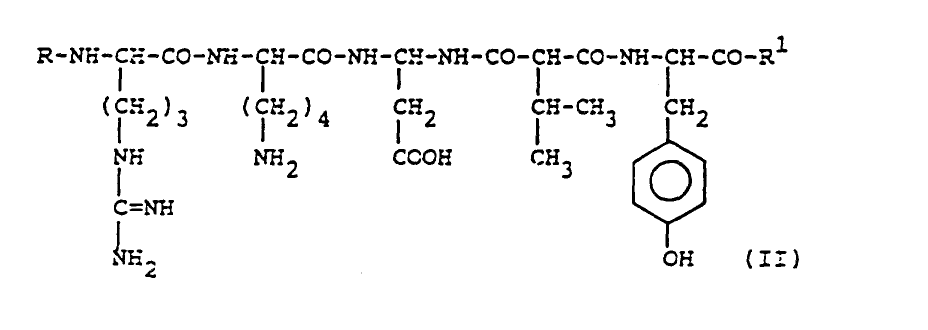 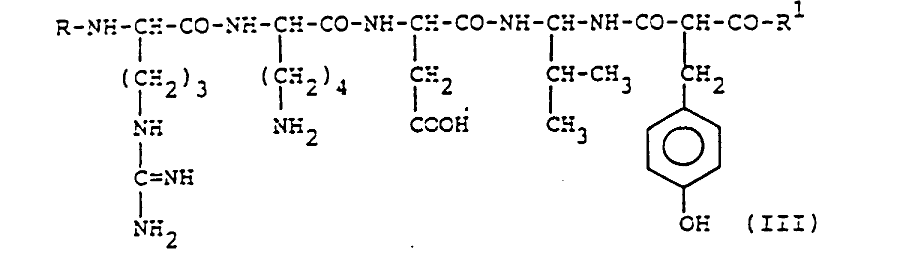 wherein R is hydrogen or an acyl radical, and
R¹ is a group of the formula -OR, wherein
R is a hydrogen atom or an alkyl radical having a straight or branched chain containing from 1 to 6 carbon atoms, an alkenyl or an alkynyl radical having a straight or branched chain containing 3-6 carbon atoms, or an aryl-alkyl or alkyl-aryl radical containing from 7 to 12 carbon atoms,
and the corresponding basic or acid addition salts pharmaceutically acceptable,
said process consisting in the removal of the protective groups from the corresponding compounds of the formulas (Ia), (IIa) and (IIIa) 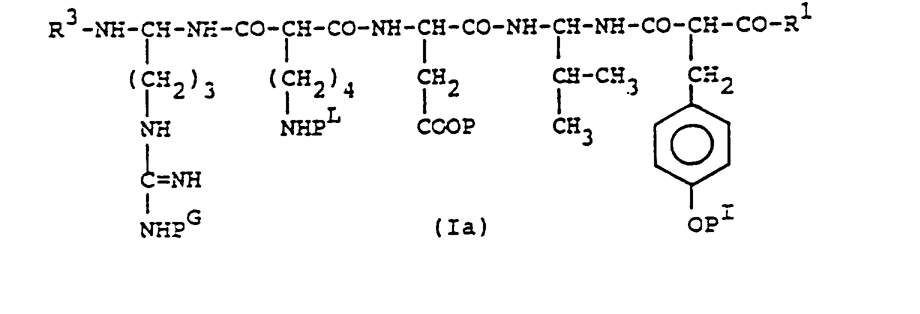 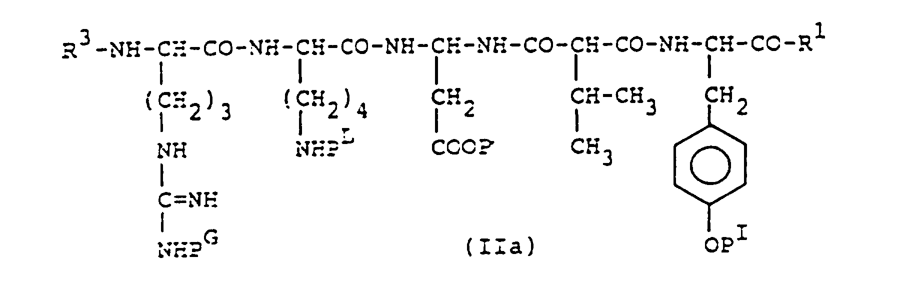 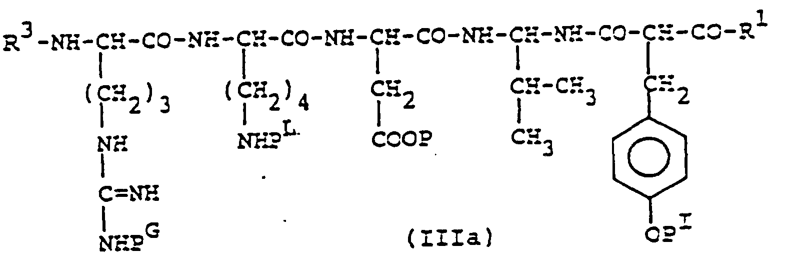 wherein:
R³ is R or a protection group of the alpha-amino function,
P^{G} has the meaning of a suitable protective group of the guanidine function,
P^{L} nas the meaning of protective group of the amino function in the side chain,
P is a protective group of the carboxyl function,
P^{I} is a protective group of the hydroxyl function of tyrosine, and
R¹ is as defined above,

9. An intermediate compound of the formulas (Ia)_{,} (IIa), (IIIa), (IV), (VI), (VII), (VIII), (IX), (X) and (XIV). 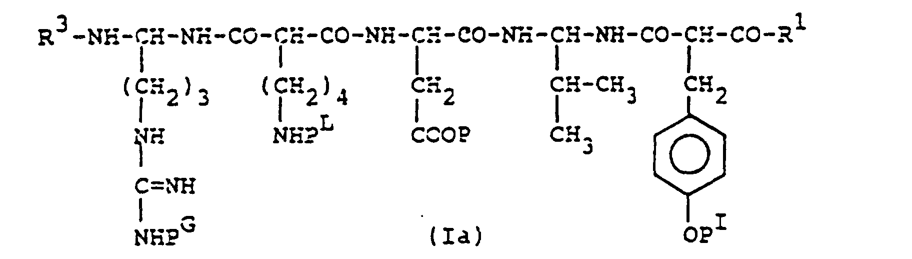 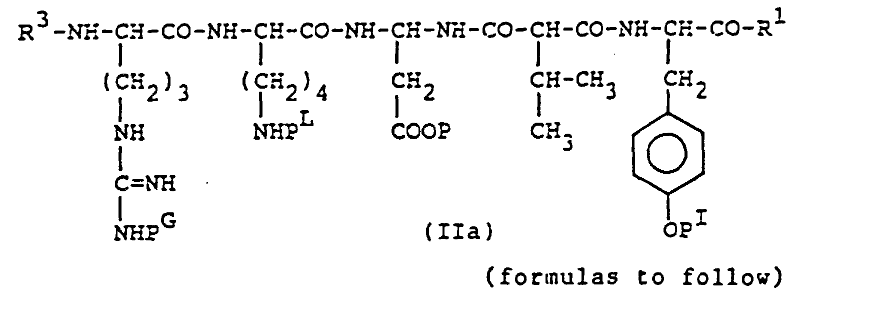 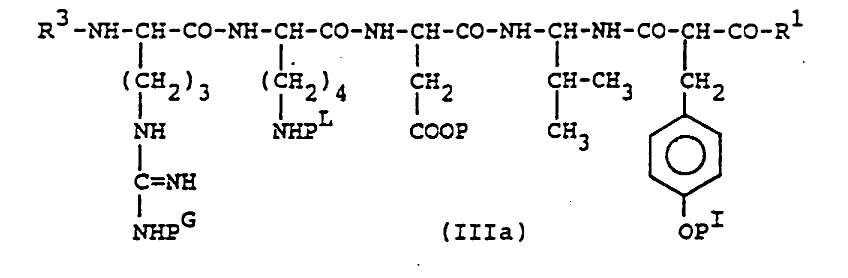 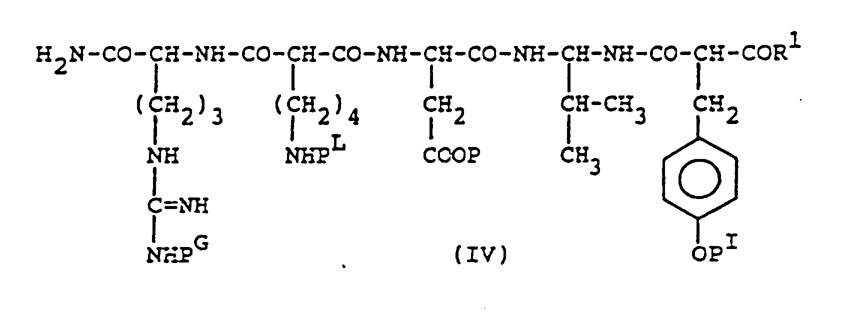 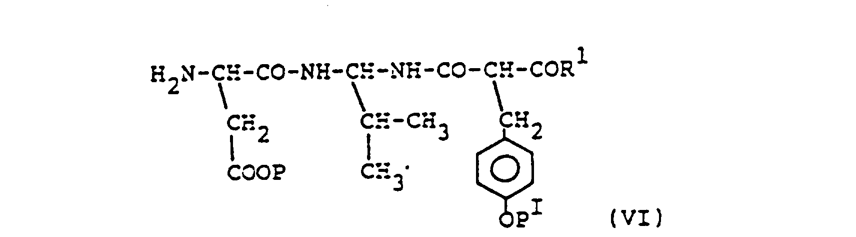 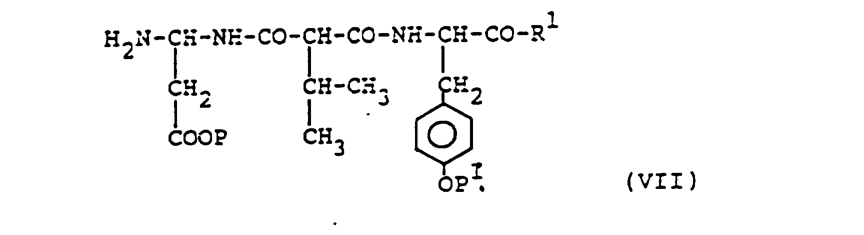 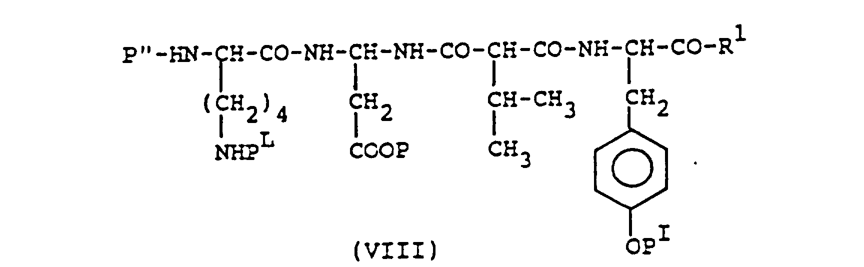 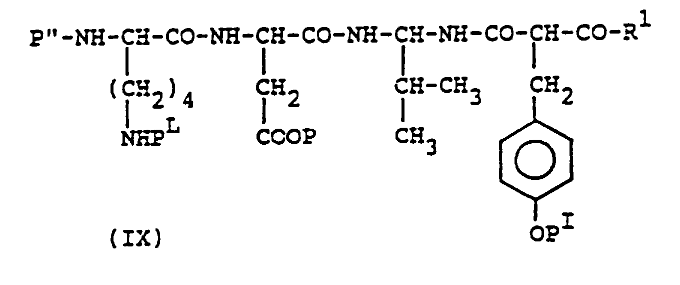 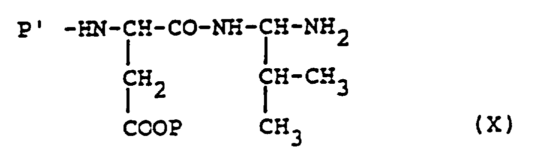 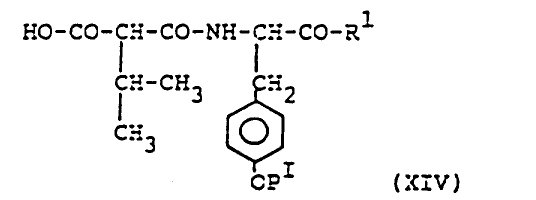 wherein
P" is hydrogen or a suitable protective group of the alpha-amino function,
P^{G} has the meaning of protective group of the guanidino function,
P^{L} has the meaning of protective group of the amino function in the side chain,
P is a protective group of the carboxyl function,
R¹ is a group of the formula -OR wherein R is as defined above, and
P^{I} is a protective group of the hydroxyl function of tyrosine.

10. An intermediate compound of the formulas (Ia), (IIa), (IIIa), (IV), (VI), (VII), (VIII), (IX), (X) and (XIV) of claim 9, wherein
P^{L} is a tert-butoxycarbonyl or a tert-amyloxycarbonyl group,
P^{G} is a benzensulfonyl group which is possibly substituted,
P is a tert-butyl, tert-amyl, benzyl or substituted benzyl group, and
P^{I} is a tert-butyl or a tert-amyl group.

## Claims (Claims for the following Contracting State(s): ES, GR)

1. A process for the preparation of a thymopentin analogue having one of the following general formulas 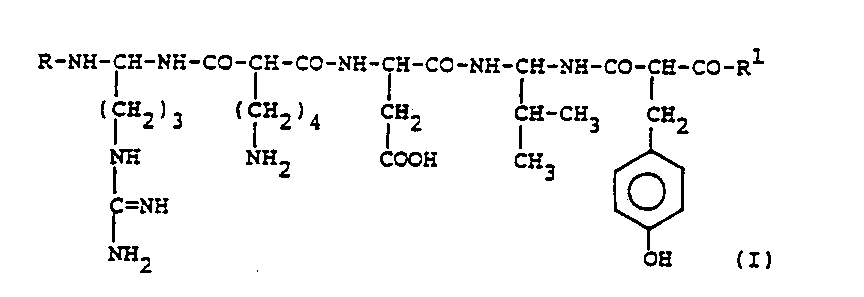 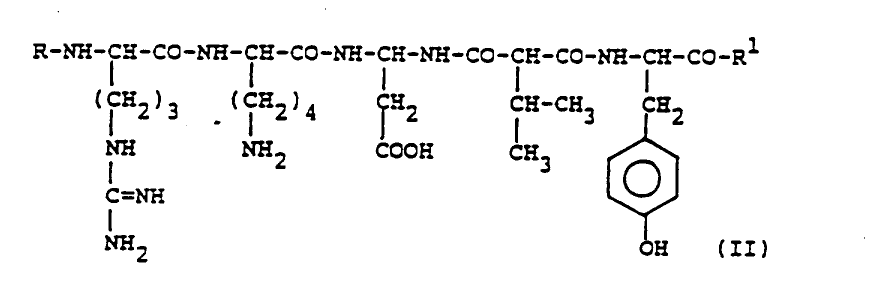 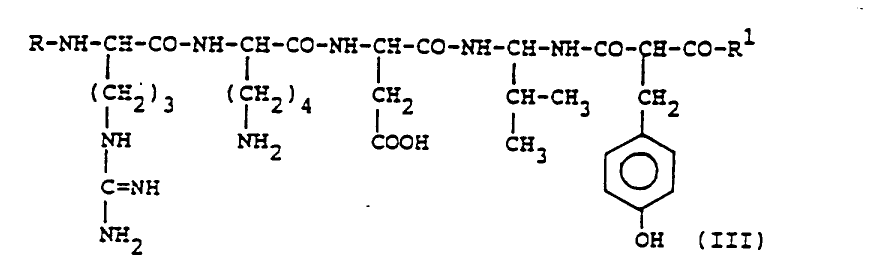 wherein R is hydrogen or a metabolically labile acyl radical or an acyl radical whose bond with the amino group is rapidly broken in the first step of the metabolic path of the product, and
R¹ is a group of the formula -OR, wherein
R is a hydrogen atom or an alkyl radical having a straight or branched chain containing from 1 to 6 carbon atoms, an alkenyl or an alkynyl radical having a straight or branched chain containing 3-6 carbon atoms, or an aryl-alkyl or alkyl-aryl radical containing from 7 to 12 carbon atoms, and the corresponding basic or acid addition salts pharmaceutically acceptable, said process consisting in the removal of the protective groups from the corresponding compounds of the formulas (Ia), (IIa) and (IIIa) 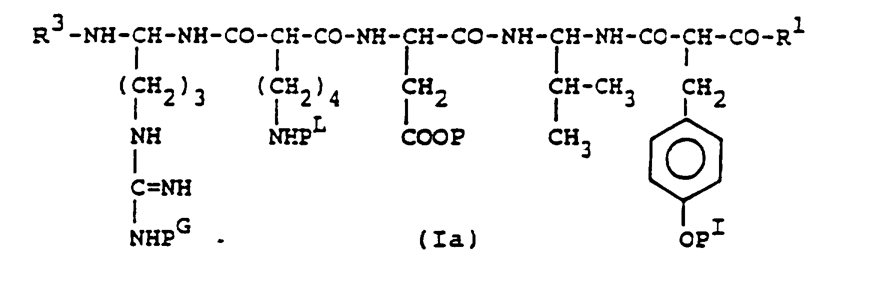 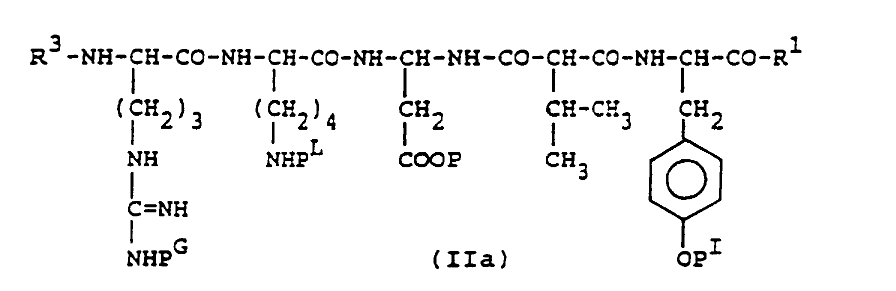 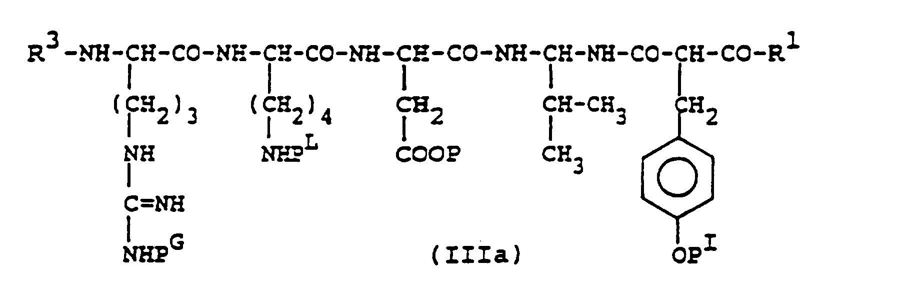 wherein:
R³ is R or protection group of the alpha-amino function,
P^{G} has the meaning of a suitable protective group of the guanidine function,
P^{L} has the meaning of the protective group of the amino function in the side chain,
P is a protective group of the carboxyl function,
P^{I} is a protective group of the hydroxyl function of tyrosine, and
R¹ is as defined above.

2. A process according to claim 1 wherein R and R¹ are as defined in claim 1 and R is hydrogen.

3. A process according to claim 2, wherein R is hydrogen.

4. A process according to claim 3, wherein the final compound is chosen in the group:
[gArg¹,(R,S)mLys,gVal⁴,(R,S)mTyr⁵]TP5,
[gAsp³,(R,S)mVal⁴]TP5, and [(R,S)mVal⁴, gTyr⁵]TP5
and the addition salts thereof.

5. A process according to claim 4, wherein the final compound is:
[gArg¹,(R,S)mLys,gVal⁴,(R,S)mTyr⁵]TP5,
and the addition salts thereof.

6. A process for the preparation of a compound of formula Ia 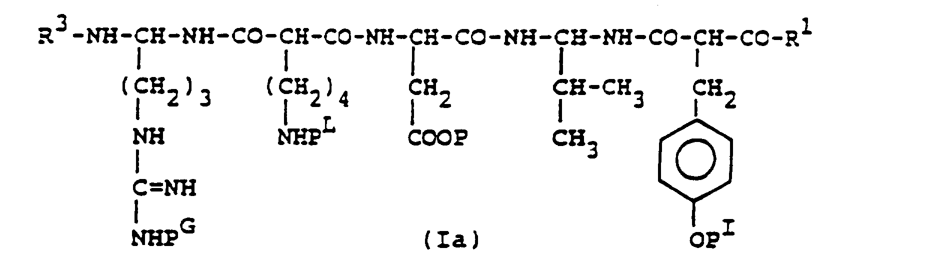 (wherein R³ = R and the other symbols are as above defined) wherein a compound of formula (IV) 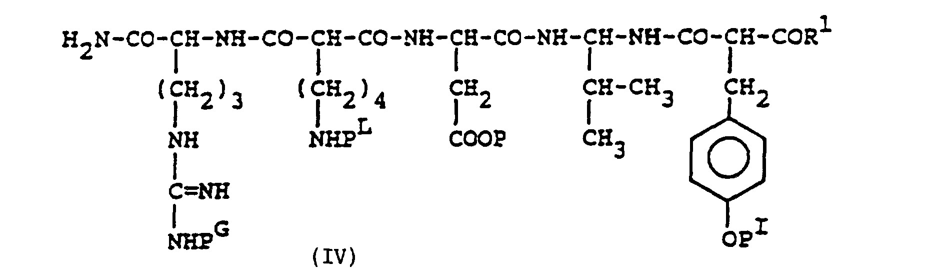 (wherein all the symbols have the above said meaning) is reacted with I,I-bis-trifluoroacetoxy-iodobenzene possobly followed by acylation of the end amino function so formed.

7. A process for the preparation of a compound of formula (IV) 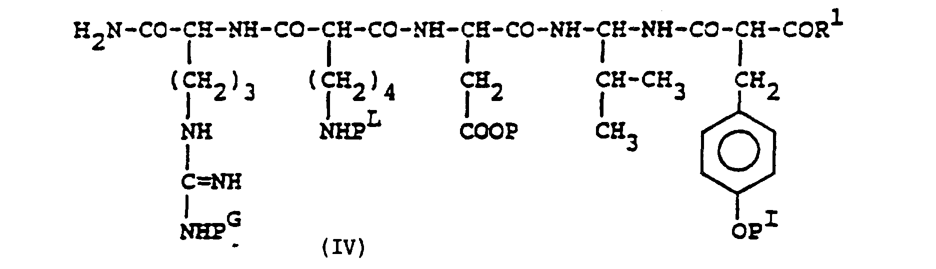 (wherein all the symbols have the above said meaning) wherein a compound of formula (V) 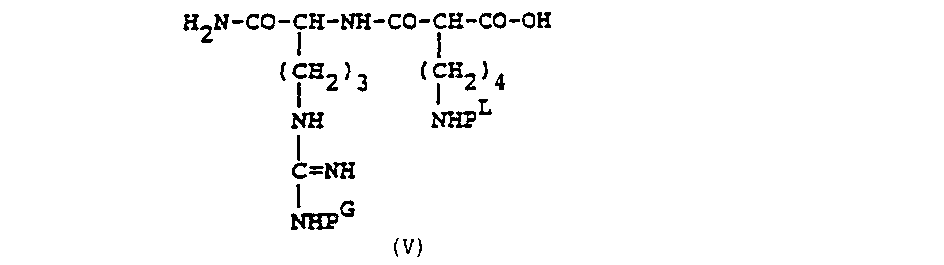 (wherein all the symbols are as already defined) is condensed with a compound of formula (VI) 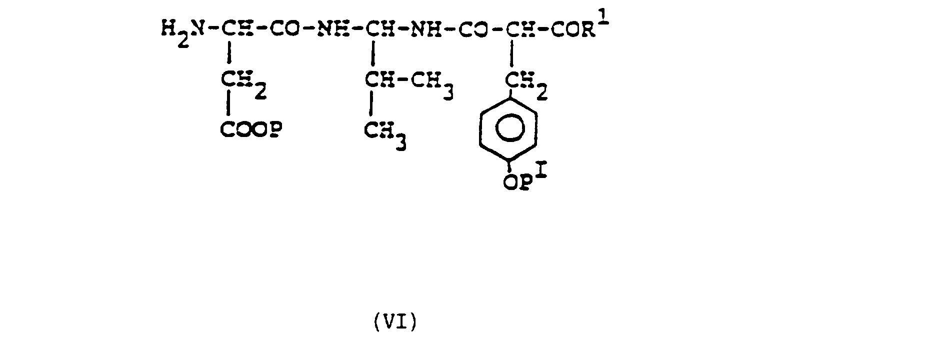 (wherein all the symbols are as already defined).

8. A process for preparing a compound of formula IIa and IIIa 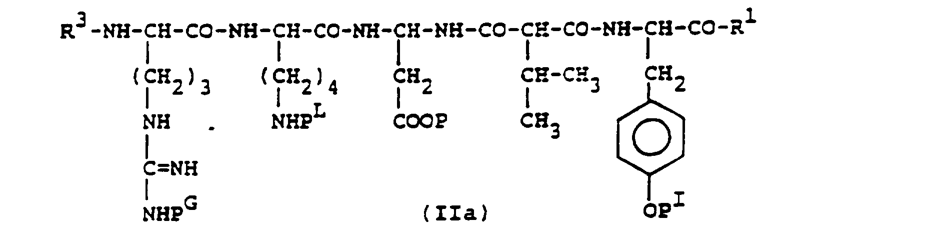 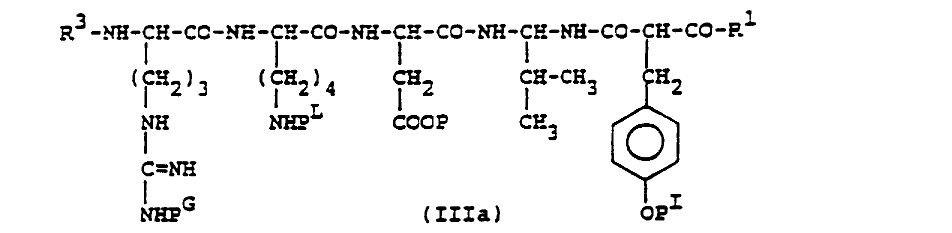 (wherein all the symbols are as above defined) wherein a compound of formula (VII) and (VI) 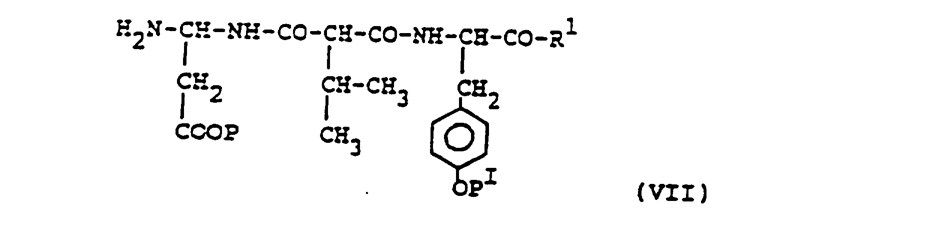 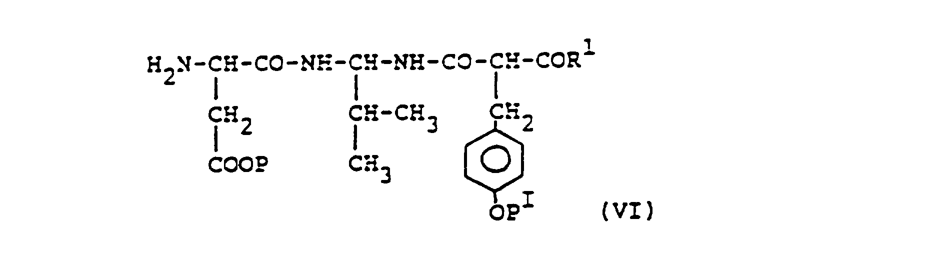 (wherein all the symbols are as above defined) are succesively condensed with a lysine and arginine residue giving the corresponding compounds of formula (VIII) and (IX) 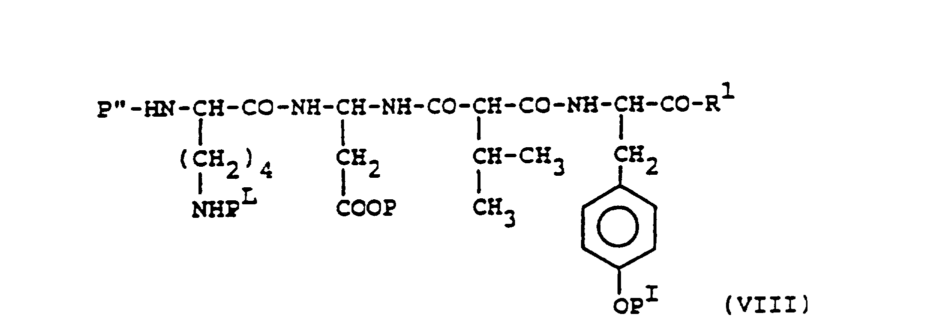 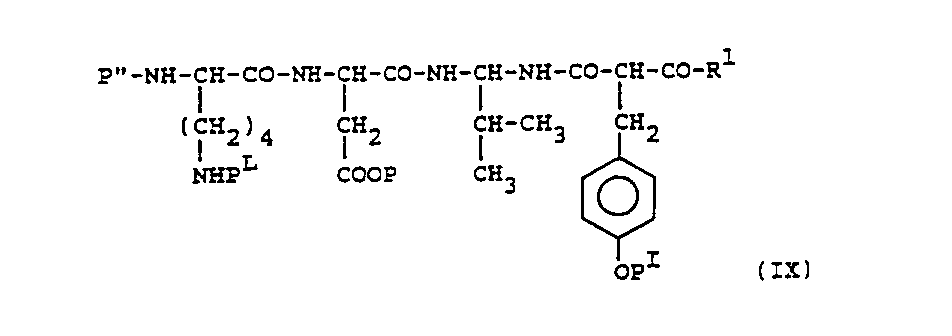 (wherein P" is H or a protective group of the alpha-amino function, which can be easily removed in conditions which do not give rise to the removal of the other protective groups and the other symbols are as above defined)

9. A process for preparing a compound of formula (VI) 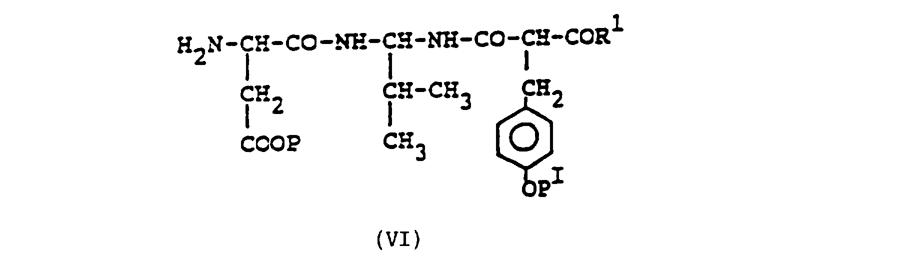 (wherein all the symbols are as above defined) wherein a dipeptide of formula (X) 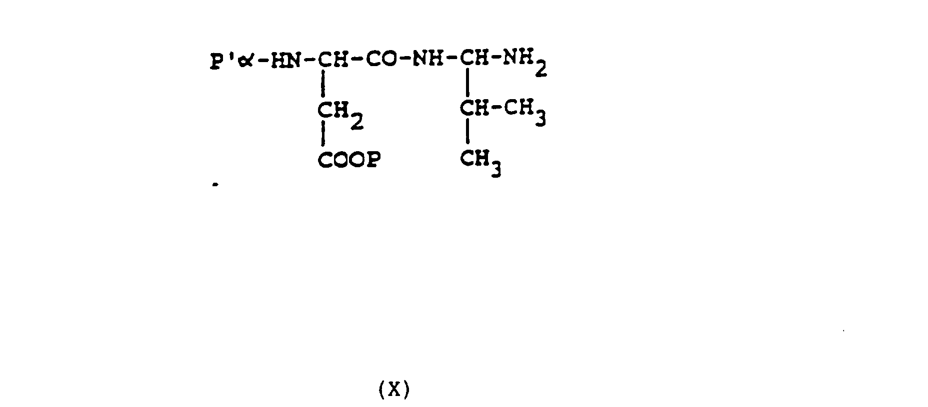 (wherein P is a protective group of the carboxyl function and P' is a protective group of the amino function in the alpha-position) is condensed with a compound of formula (XI) 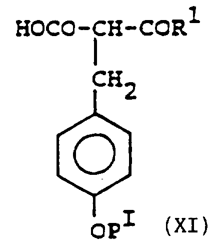 wherein R¹ and P^{I} are as above defined.

10. A process for preparing a compound of formula (VIII) 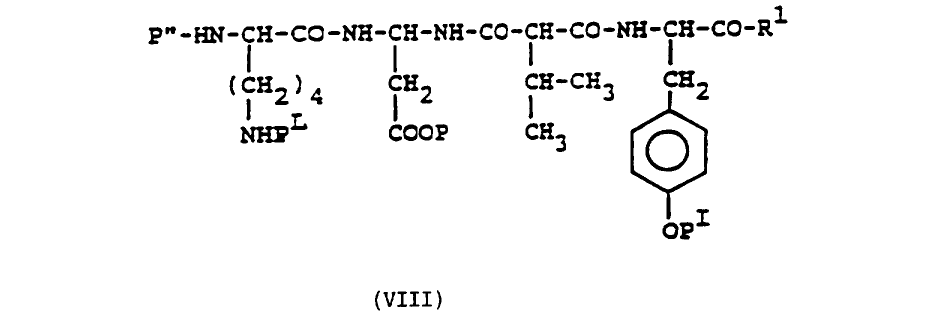 (wherein all the symbols are as above defined) wherein an amide of formula of formula (XIII) 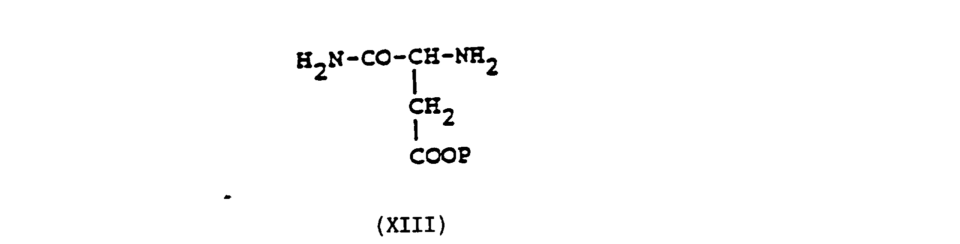 (wherein all the symbols are as above defined) is condensed with a compound of formula (XIV) 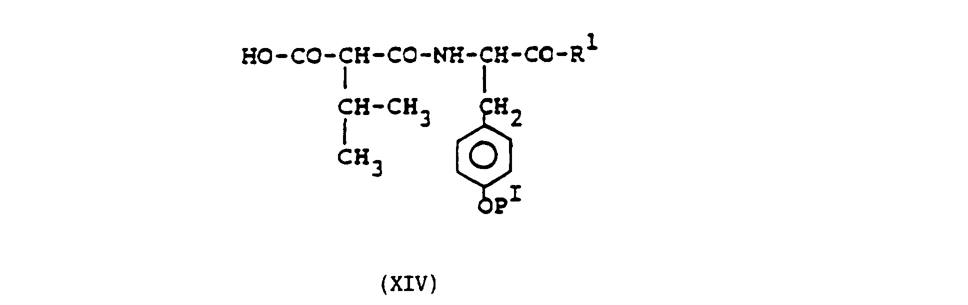 (wherein all the symbols are as above defined) followed by the conversion of the amide group into the primary amino group by treatment with TIB.

11. A process for the preparation of a compound of formula (XIV) 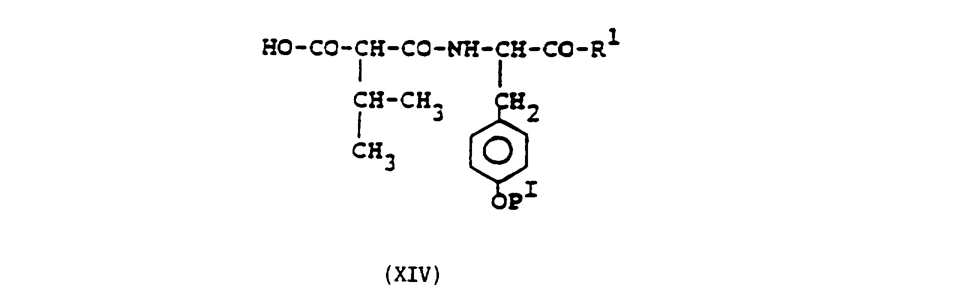 (wherein all the symbols are as above defined) wherein a Meldrum acid of formula (XV) 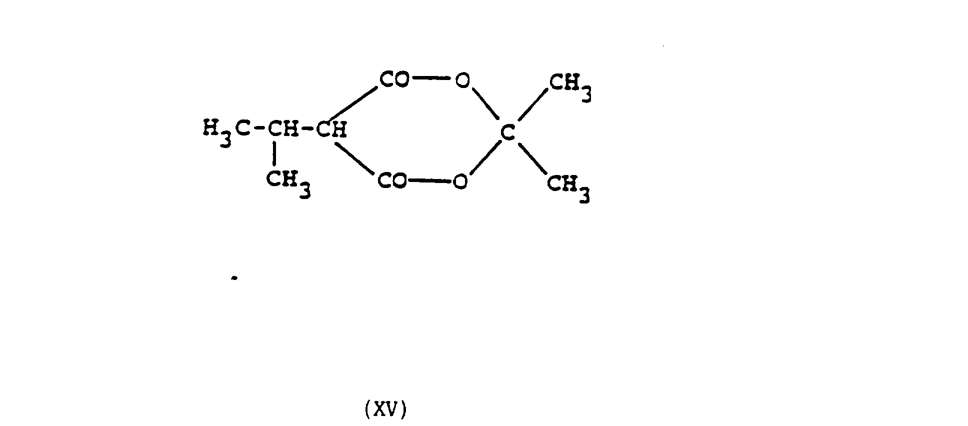 is condensed with a tyrosine opportunely protected of formula (XVI) 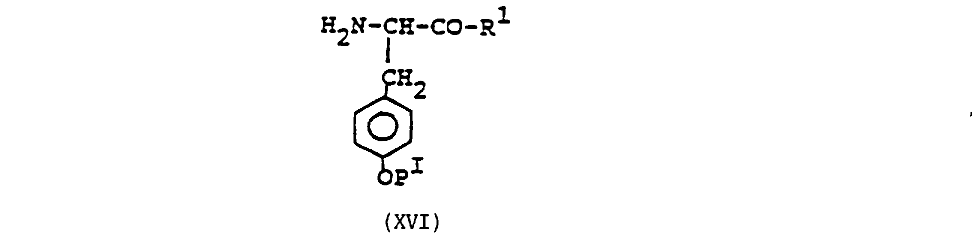 (wherein all the symbols are as above defined) in the presence of a silanizing agent.

12. A process according to claims 6 - 11 wherein:
P^{L} is a tert-butoxycarbonyl or a tert-amyloxycarbonyl group,
P^{G} is a benzensulfonyl group which is possibly substituted,
P is a tert-butyl, tert-amyl, benzyl or substituted benzyl group and
P^{I} is a tert-butyl or a tert-amyl group.

13. A process for the preparation of a pharmaceutical composition wherein an active compound as obtained according to the process of claim 1 is combined with a pharmaceutically acceptable inert vehicle and possibly with some standard additives accordingly chosen.

## Patentansprüche (Patentansprüche für folgende(n) Vertragsstaat(en): AT, BE, CH, DE, DK, FR, GB, LI, LU, NL, SE)

1. Thymopentin-Analoges mit einer der folgenden Formeln 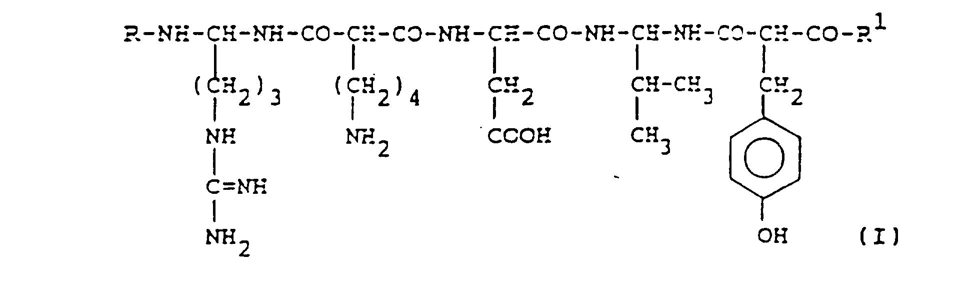 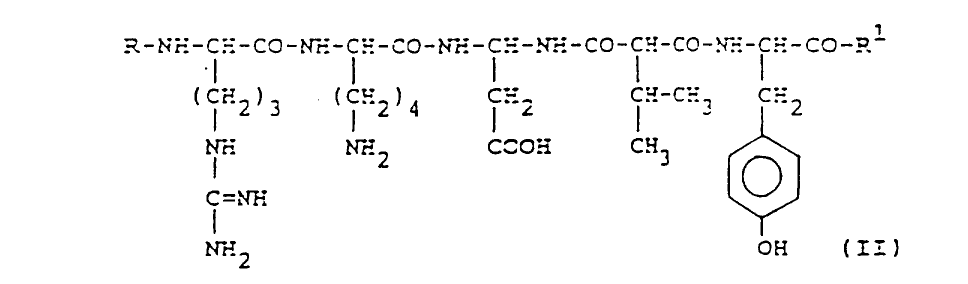 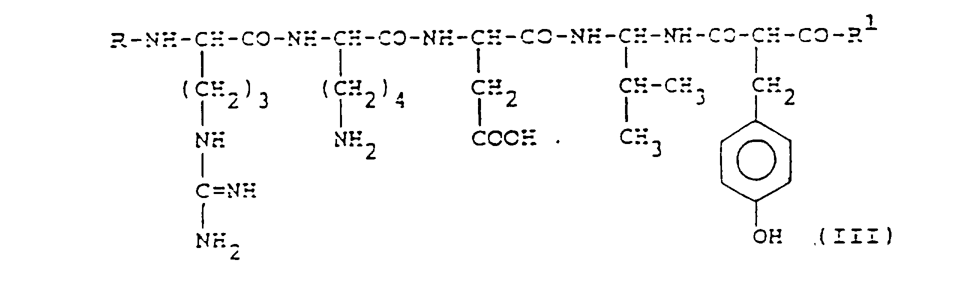
worin R Wasserstoff oder einen metabolisch labilen Acylrest oder einen Acylrest bedeutet, dessen Bindung mit der Aminogruppe in den ersten Stufen des Stoffwechselwegs des Produkts leicht aufgebrochen wird, und R¹ eine Gruppe der Formel -OR bedeutet,
worin R ein Wasserstoffatom oder einen Alkylrest mit einer geraden oder verzweigten Kette mit 1 bis 6 Kohlenstoffatomen, einen Alkenyl- oder Alkinylrest mit einer geraden oder verzweigten Kette mit 3 bis 6 Kohlenstoffatomen oder einen Arylalkyl- oder Alkylarylrest mit 7 bis 12 Kohlenstoffatomen bedeutet, sowie die entsprechenden pharmazeutisch verträglichen Säure- oder Basenadditionssalze.

2. Verbindung nach Anspruch 1, wobei R und R¹ die in Anspruch 1 definierte Bedeutung haben und R Wasserstoff bedeutet.

3. Verbindung nach Anspruch 2, worin R Wasserstoff bedeutet.

4. Verbindung nach Anspruch 3, ausgewählt unter
[gArg¹,(R,S)mLys,gVal⁴, (R,S)mTyr⁵]TP5,
[gAsp³, (R,S)mVal⁴]TP5, [(R,S)mVal⁴,gTyr⁵]TP5,
und den Säureadditionssalzen davon.

5. Verbindung nach Anspruch 4, ausgewählt unter
[gArg¹,(R,S)mLys,gVal⁴,(R,S)mTyr⁵]TP5
und den Additionssalzen davon.

6. Pharmazeutische Zusammensetzung, umfassend eine Verbindung nach Anspruch 1 als Wirkstoff.

7. Verbindung nach Anspruch 1 zur Verwendung als Arzneistoff.

8. Verfahren zur Herstellung eines Thymopentin-Analogen mit einer der folgenden Formeln 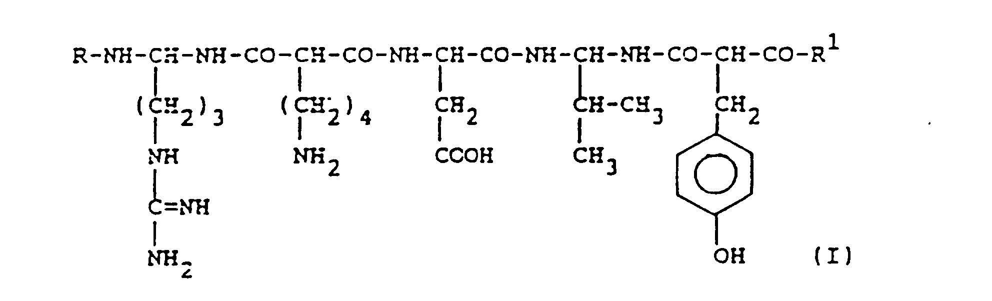 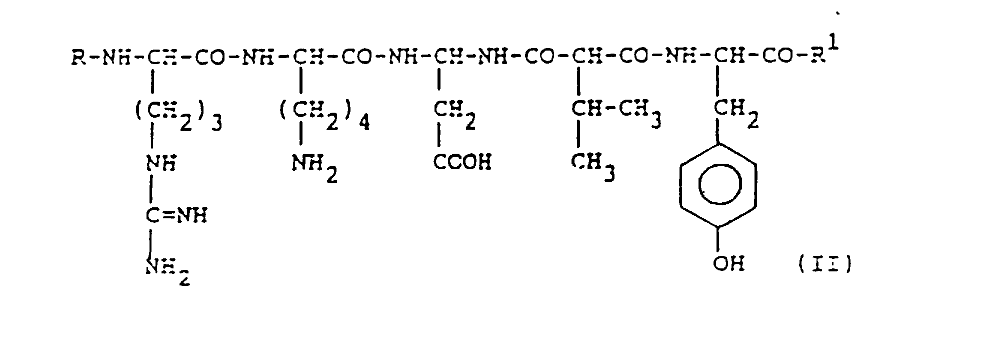 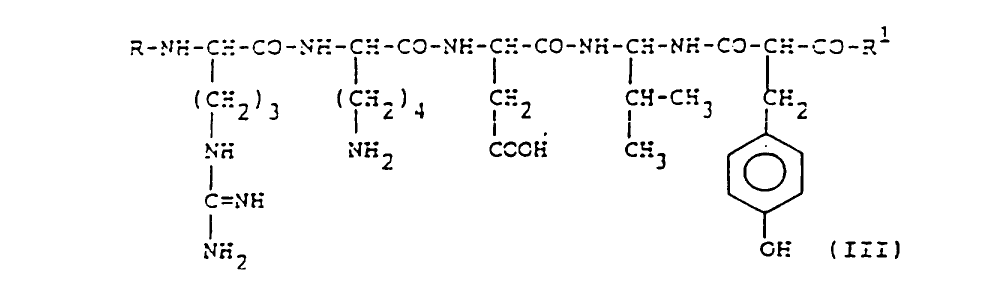
worin R ein Wasserstoffatom oder einen Acylrest bedeutet und
R¹ eine Gruppe der Formel -OR bedeutet,
worin R ein Wasserstoffatom oder einen Alkylrest mit einer geraden oder verzweigten Kette mit 1 bis 6 Kohlenstoffatomen, einen Alkenyl- oder einen Alkinylrest mit einer geraden oder verzweigten Kette mit 3 bis 6 Kohlenstoffatomen oder einen Arylalkyl- oder Alkylarylrest mit 7 bis 12 Kohlenstoffatomen bedeutet, und der entsprechenden pharmazeutisch verträglichen Basen- oder Säureadditionssalze, wobei das Verfahren in der Entfernung der Schutzgruppen von den entsprechenden Verbindungen der Formeln (Ia), (IIa) und (IIIa) besteht 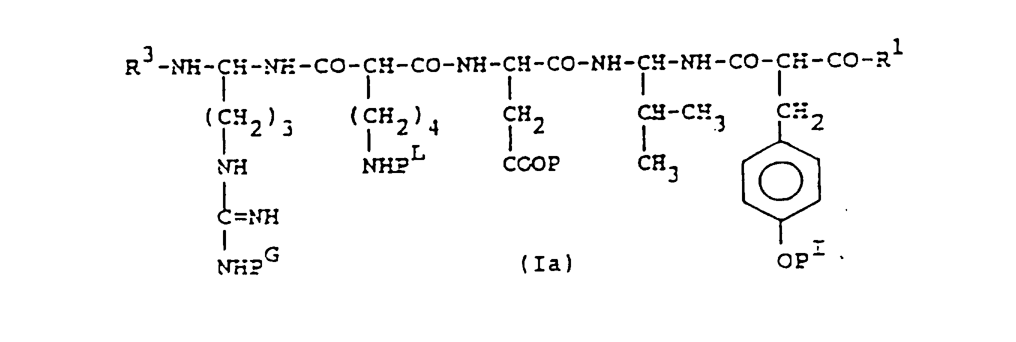 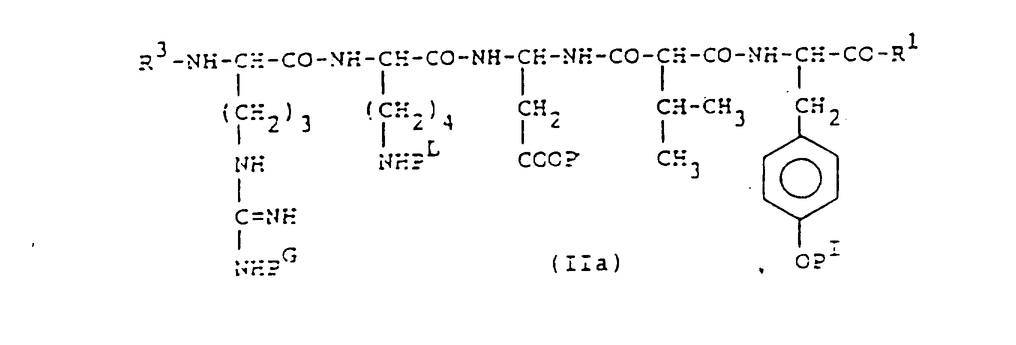 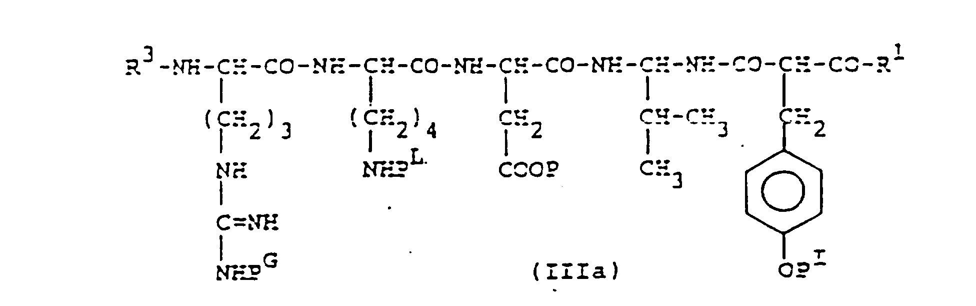 wobei
R³ R oder eine Schutzgruppe für die α-Aminofunktion bedeutet,
P^{G} eine geeignete Schutzgruppe für die Guanidinfunktion bedeutet,
P^{L} eine Schutzgruppe für die Aminofunktion in der Seitenkette bedeutet,
P eine Schutzgruppe für die Carboxylfunktion bedeutet,
P^{I} eine Schutzgruppe für die Hydroxylfunktion von Tyrosin bedeutet und
R¹ die vorstehend definierte Bedeutung hat.

9. Zwischenprodukt der Formeln (Ia), (IIa), (IIIa), (IV), (VI), (VII), (VIII), (IX), (X) und (XIV) 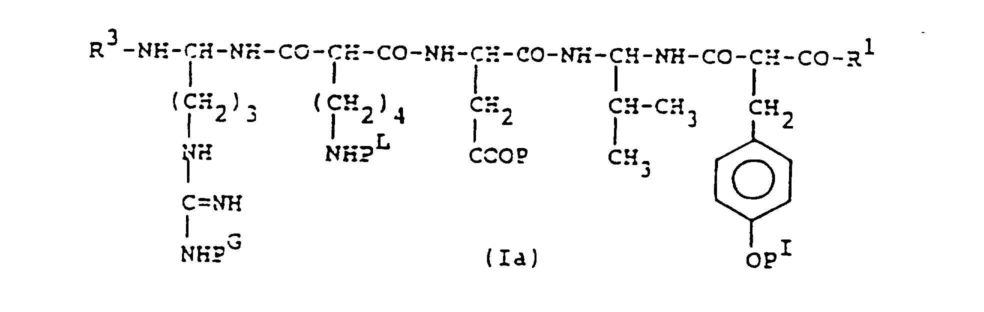 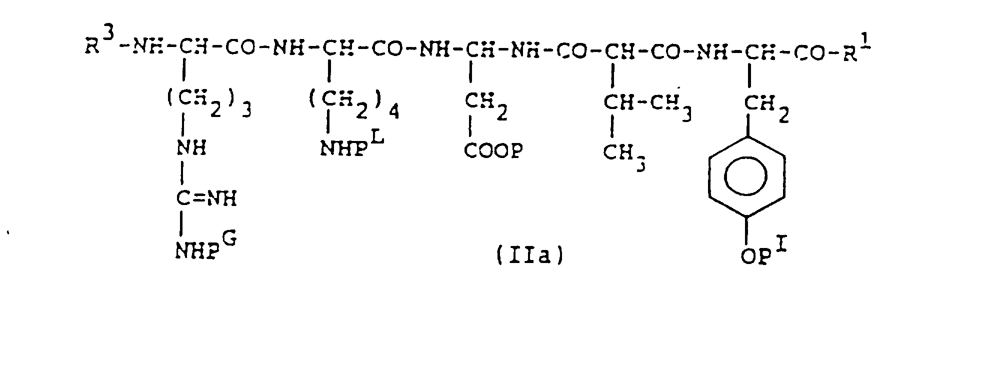 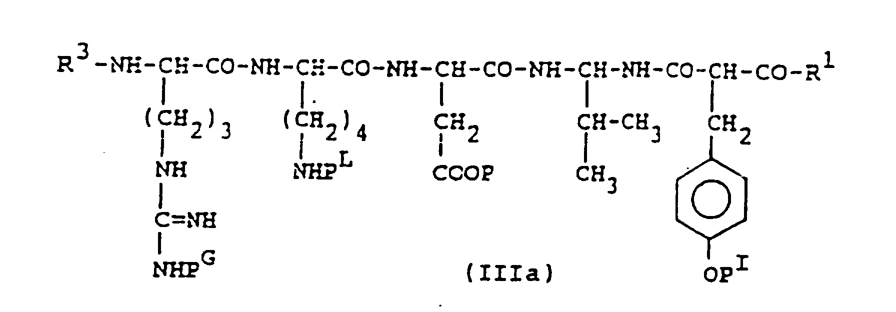 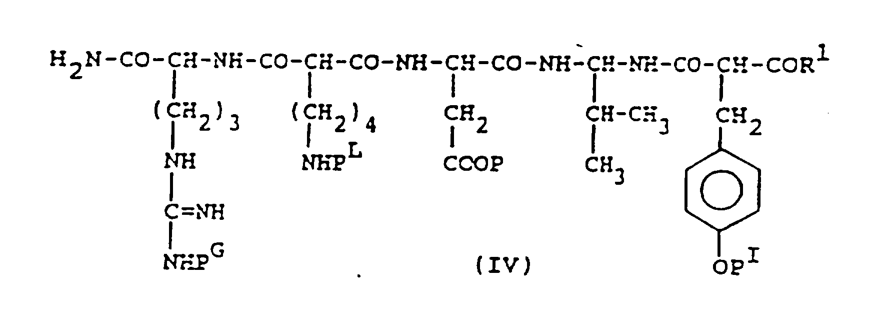 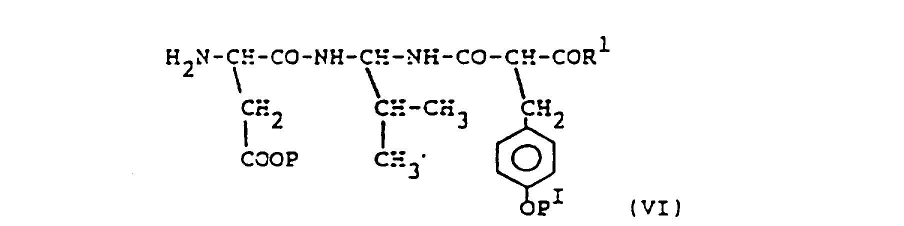 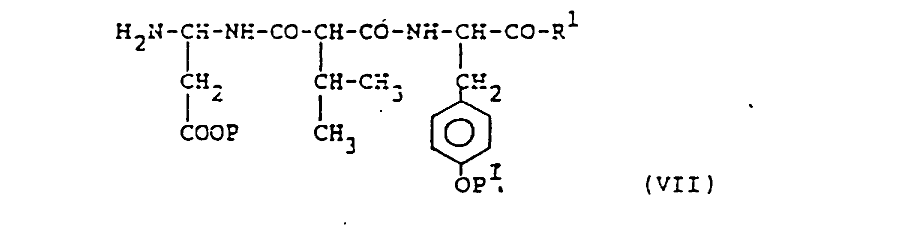 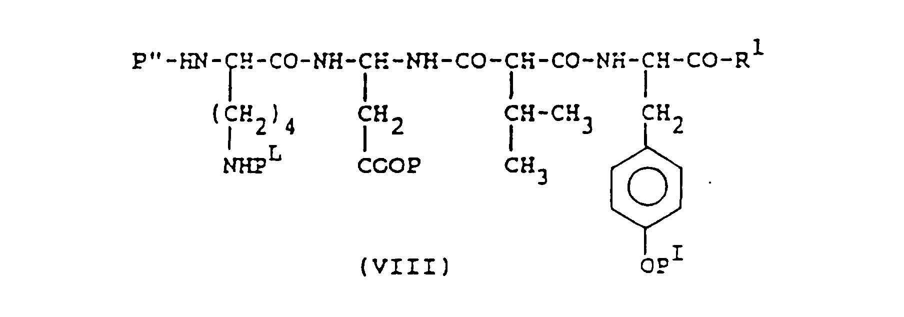 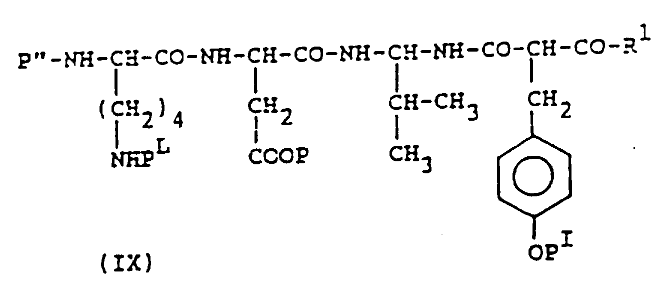 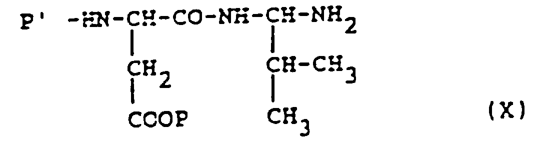 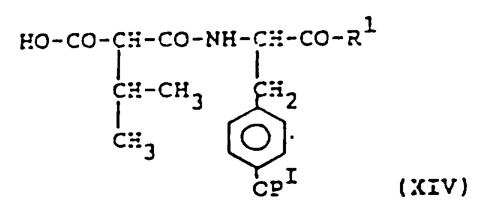 wobei
P" Wasserstoff oder eine geeignete Schutzgruppe für die α-Aminofunktion bedeutet,
P^{G} eine Schutzgruppe für die Guanidinofunktion bedeutet,
P^{L} eine Schutzgruppe für die Aminofunktion in der Seitenkette bedeutet,
P eine Schutzgruppe für die Carboxylfunktion bedeutet,
R¹ eine Gruppe der Formel -OR bedeutet, worin R die vorstehend definierte Bedeutung hat und
P^{I} eine Schutzgruppe für die Hydroxylfunktion von Tyrosin bedeutet.

10. Zwischenprodukt der Formeln (Ia), (IIa), (IIIa), (IV), (VI), (VII), (VIII), (IX), (X) und (XIV) nach Anspruch 9, wobei
P^{L} eine tert.-Butoxycarbonyl- oder tert.-Amyloxycarbonylgruppe bedeutet,
P^{G} eine Benzylsulfonylgruppe, die ggf. substituiert ist, bedeutet,
P eine tert.-Butyl-, tert.-Amyl-, Benzyl- oder substituierte Benzylgruppe bedeutet, und
P^{I} eine tert.-Butyl- oder tert.-Amylgruppe bedeutet.

## Patentansprüche (Patentansprüche für folgende(n) Vertragsstaat(en): ES, GR)

1. Verfahren zur Herstellung eines Thymopentin-Analogen mit einer der folgenden allgemeinen Formeln 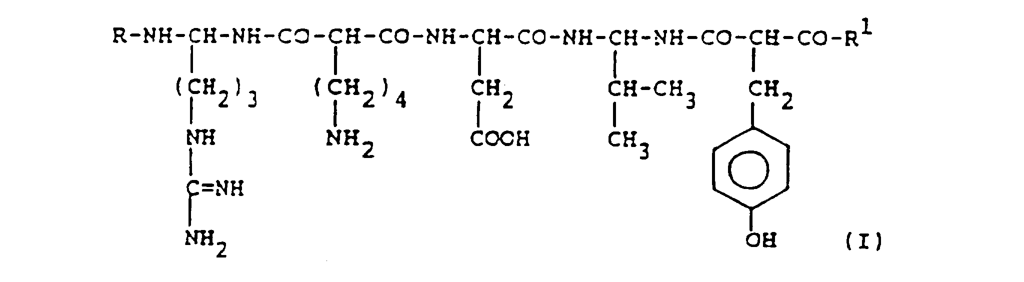 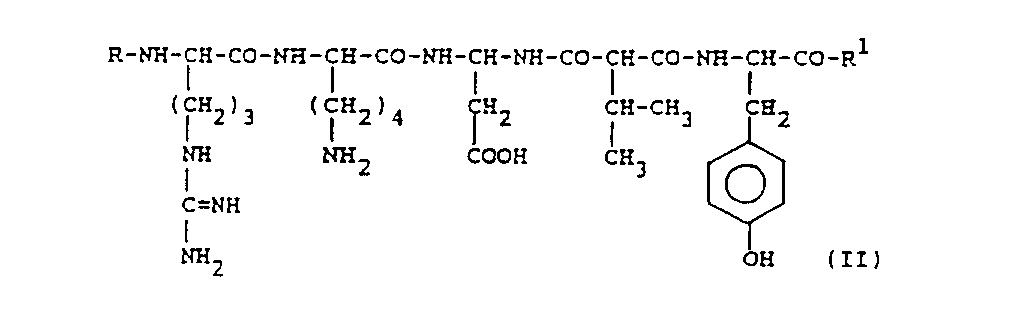 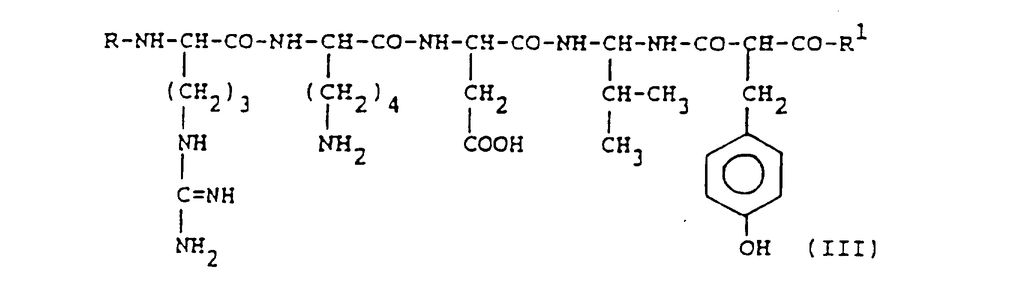
worin R Wasserstoff oder einen metabolisch labilen Acylrest oder einen Acylrest bedeutet, dessen Bindung mit der Aminogruppe in den ersten Stufen des Stoffwechselwegs des Produkts leicht aufgebrochen wird, und R¹ eine Gruppe der Formel -OR bedeutet,
worin R ein Wasserstoffatom oder einen Alkylrest mit einer geraden oder verzweigten Kette mit 1 bis 6 Kohlenstoffatomen, einen Alkenyl- oder Alkinylrest mit einer geraden oder verzweigten Kette mit 3 bis 6 Kohlenstoffatomen oder einen Arylalkyl- oder Alkylarylrest mit 7 bis 12 Kohlenstoffatomen bedeutet, sowie die entsprechenden pharmazeutisch verträglichen Säure- oder Basenadditionssalze, wobei das Verfahren in der Entfernung der Schutzgruppen von den entsprechenden Verbindungen der Formeln (Ia), (IIa) und (IIIa) besteht 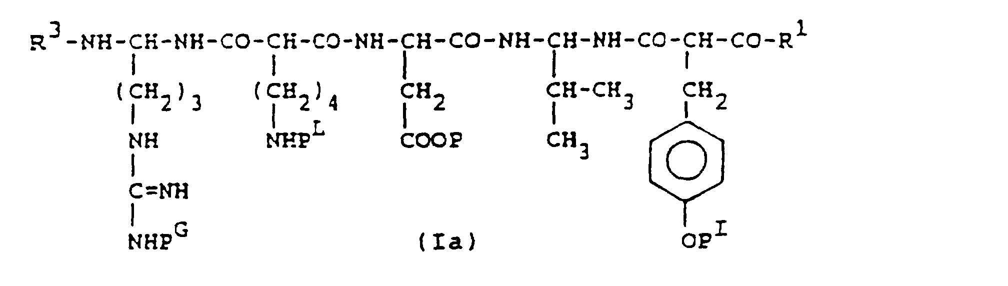 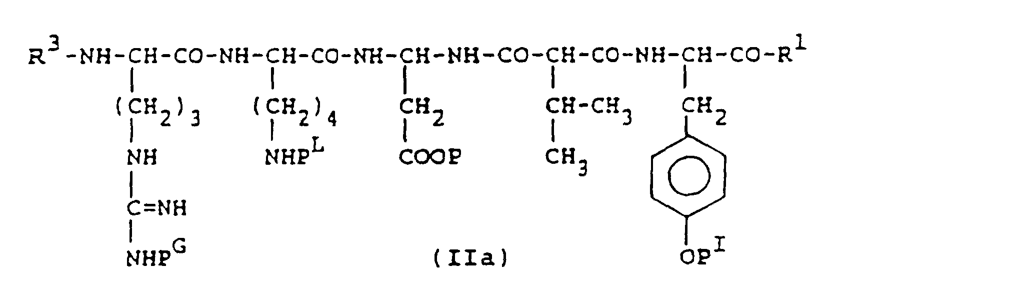 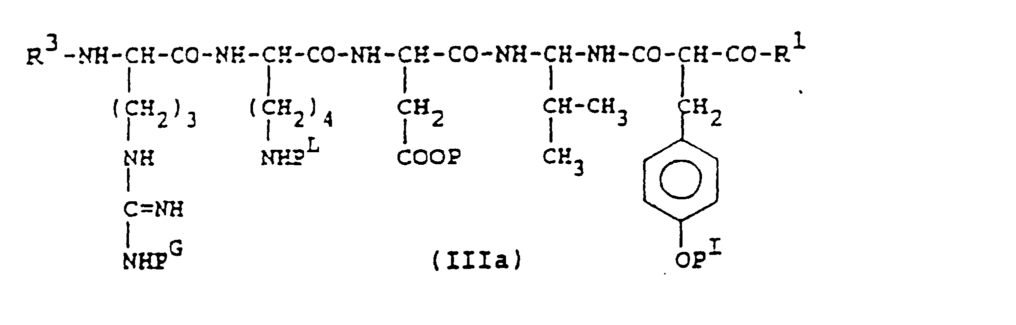 wobei
R³ R oder eine Schutzgruppe für die α-Aminofunktion bedeutet,
P^{G} eine geeignete Schutzgruppe für die Guanidinfunktion bedeutet,
P^{L} eine Schutzgruppe für die Aminofunktion in der Seitenkette bedeutet,
P eine Schutzgruppe für die Carboxylfunktion bedeutet,
P^{I} eine Schutzgruppe für die Hydroxylfunktion von Tyrosin bedeutet und
R¹ die vorstehend definierte Bedeutung hat.

2. Verfahren nach Anspruch 1, wobei R und R¹ die in Anspruch 1 definierte Bedeutung haben und R Wasserstoff bedeutet.

3. Verfahren nach Anspruch 2, worin R Wasserstoff bedeutet.

4. Verfahren nach Anspruch 3, worin das Endprodukt aus der Gruppe
[gArg¹, (R,S)mLys,gVal⁴, (R,S)mTyr⁵]TP5,
[gAsp³, (R,S)mVal⁴]TP5, [(R,S)mVal⁴,gTyr⁵]TP5,
und den Säureadditionssalzen davon ausgewählt ist.

5. Verfahren nach Anspruch 4, worin das Endprodukt unter
[gArg¹,(R,S)mLys,gVal⁴,(R,S)mTyr⁵]TP5
und den Additionssalzen davon ausgewählt ist.

6. Verfahren zur Herstellung einer Verbindung der Formel Ia 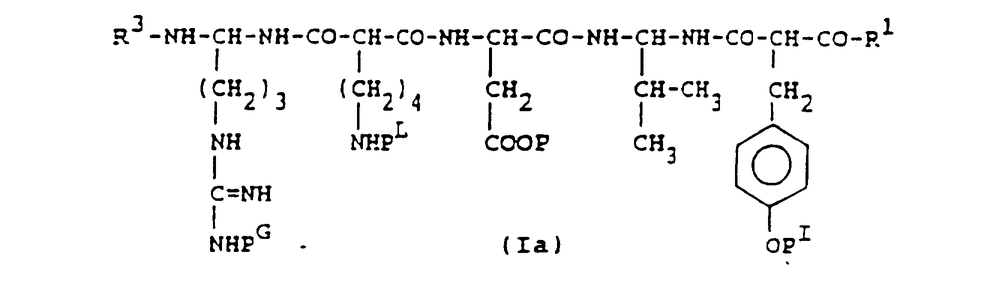
(worin R³ = R und die anderen Symbole die vorstehend definierte Bedeutung haben), wobei eine Verbindung der Formel (IV) 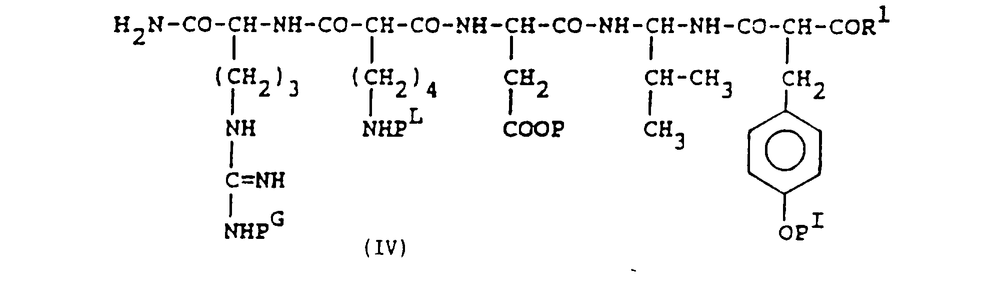
(worin alle Symbole die vorstehend definierte Bedeutung haben) mit I,I-Bis-trifluoroacetoxy-iodbenzol umgesetzt wird, wonach sich ggf. eine Acylierung der gebildeten Endaminofunktion anschließt.

7. Verfahren zur Herstellung einer Verbindung der Formel (IV) 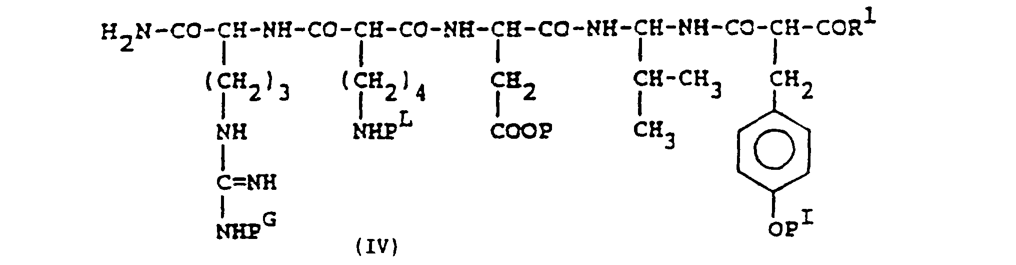
(worin alle Symbole die vorstehend definierte Bedeutung haben), wobei eine Verbindung der Formel (V) 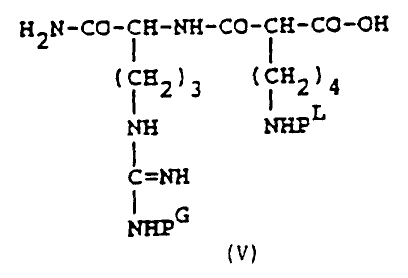
(worin alle Symbole die bereits definierte Bedeutung haben) mit einer Verbindung der Formel (VI) kondensiert wird 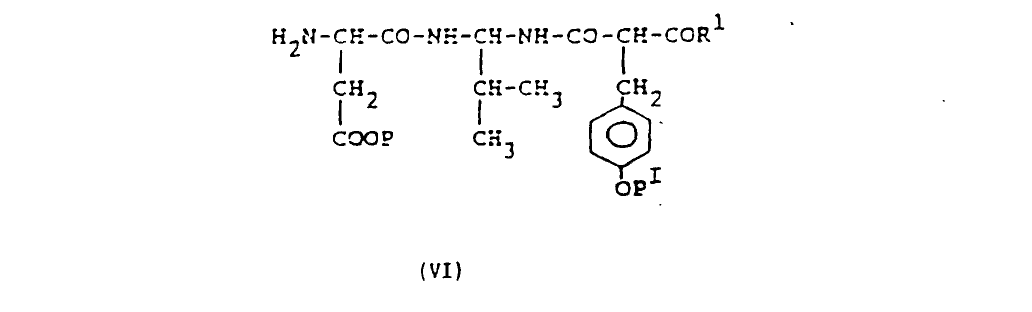 (worin alle Symbole die vorstehend definierte Bedeutung haben).

8. Verfahren zur Herstellung einer Verbindung der Formeln IIa und IIIa 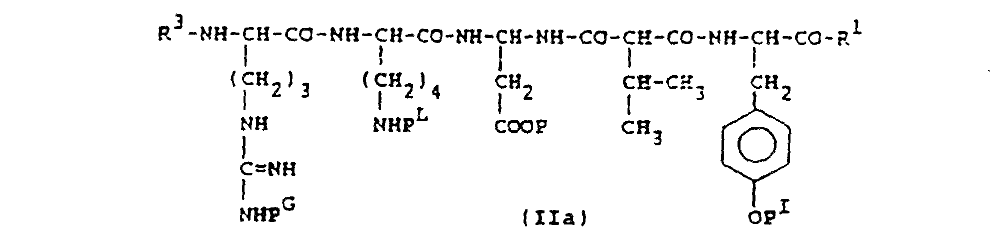 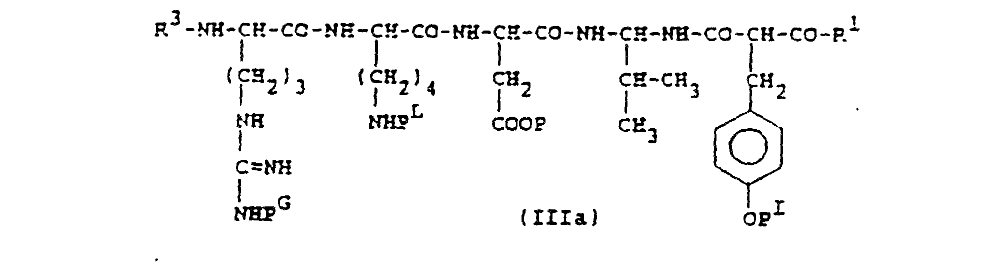
(worin alle Symbole die vorstehend definierte Bedeutung haben), wobei eine Verbindung der Formeln (VII) und (VI) 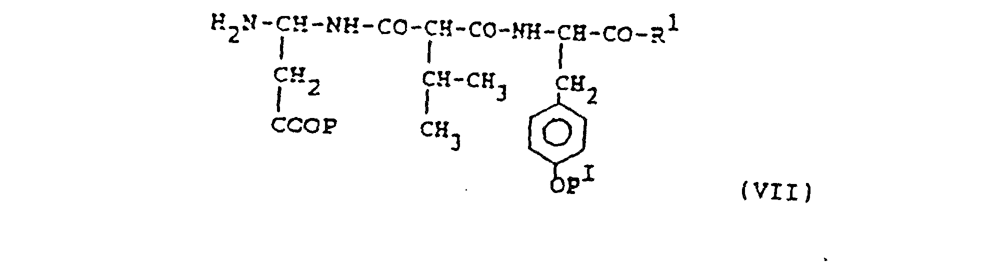 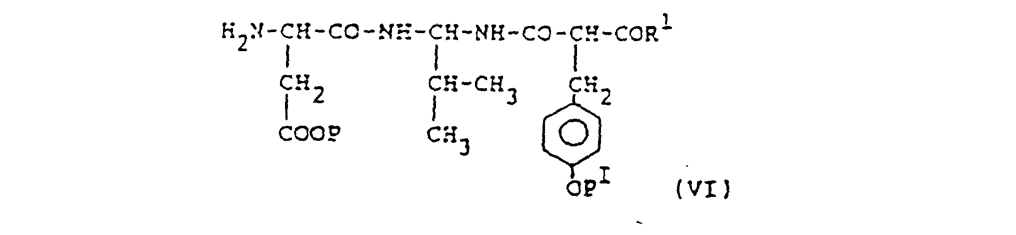
(worin alle Symbole die vorstehend definierte Bedeutung haben) nacheinander mit einem Lysin- und Argininrest unter Bildung der entsprechenden Verbindungen der Formeln (VIII) und (IX) kondensiert werden 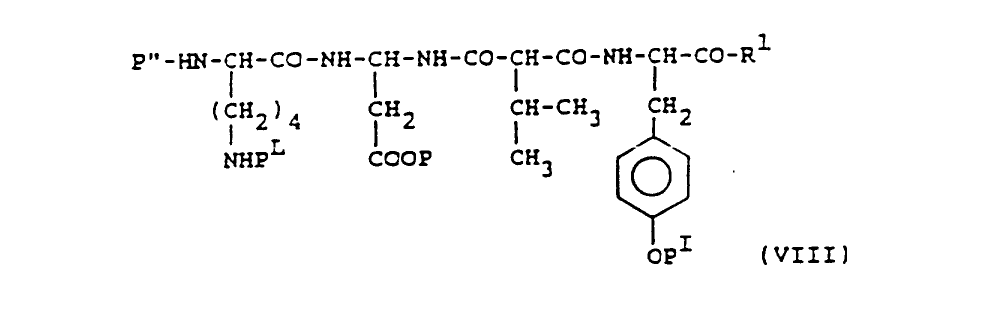 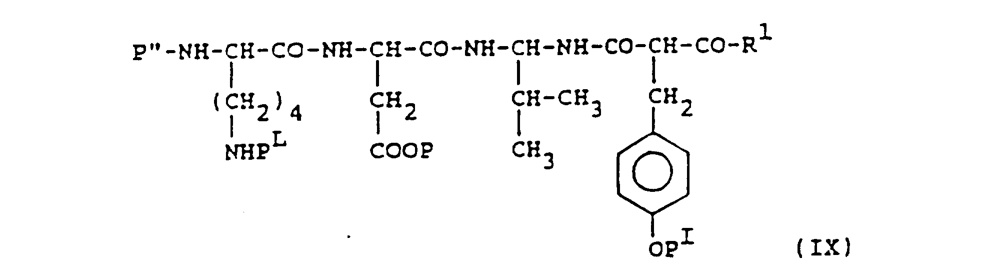
(worin P" Wasserstoff oder eine Schutzgruppe für die α-Aminofunktion bedeutet, die unter Bedingungen, die keine Entfernung der anderen Schutzgruppen hervorruft, leicht entfernt werden kann, wobei die übrigen Symbole die vorstehend definierte Bedeutung haben).

9. Verfahren zur Herstellung einer Verbindung der Formel (VI) 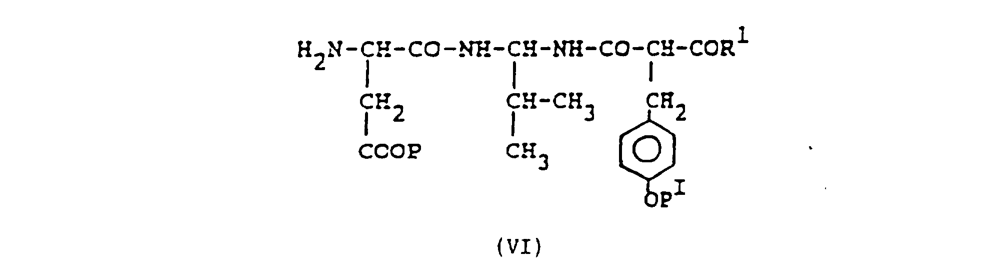
worin alle Symbole die vorstehend definierte Bedeutung haben), worbei ein Dipeptid der Formel (X) 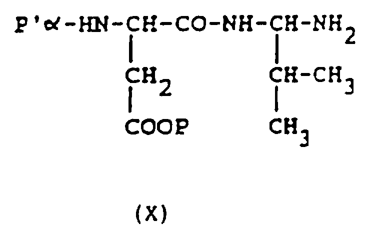
(worin P eine Schutzgruppe für die Carboxylfunktion und P' eine Schutzgruppe für die Aminofunktion in der α-Stellung bedeuten), mit einer Verbindung der Formel (XI) kondensiert wird 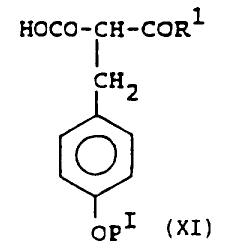 worin R¹ und P^{I} die vorstehend definierte Bedeutung haben.

10. Verfahren zur Herstellung einer Verbindung der Formel (VIII) 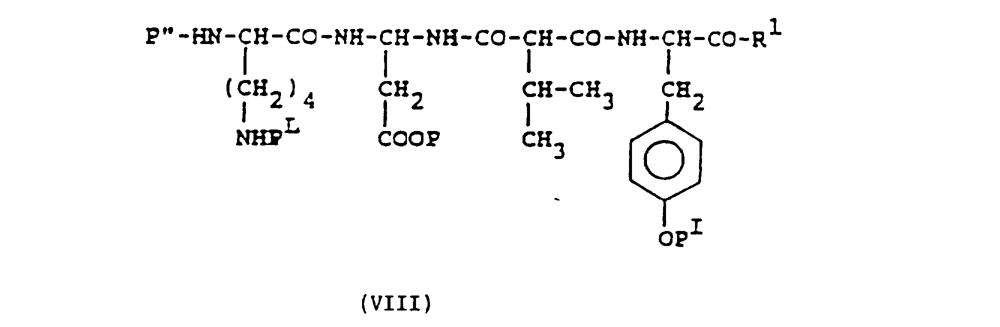
(worin alle Symbole die vorstehend definierte Bedeutung haben), wobei ein Amid der Formel (XIII) 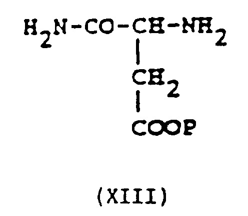
(worin alle Symbole die vorstehend definierte Bedeutung haben), mit einer Verbindung der Formel (XIV) kondensiert wird 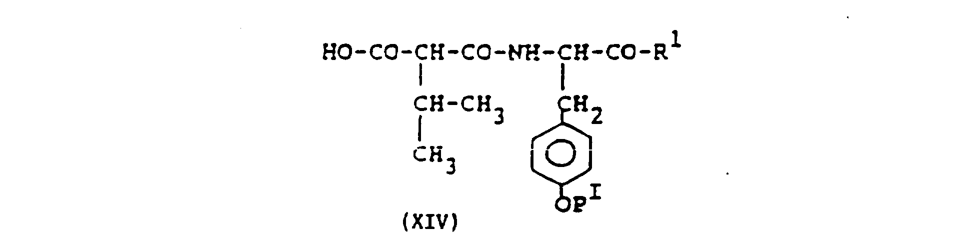
(worin alle Symbole die vorstehend definierte Bedeutung haben), wonach sich eine Umwandlung der Amidgruppe in eine primäre Aminogruppe durch Behandlung mit TIB anschließt.

11. Verfahren zur Herstellung einer Verbindung der Formel (XIV) 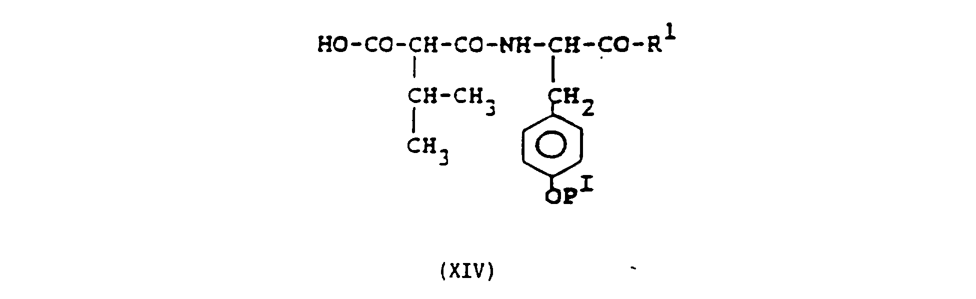
(worin alle Symbole die vorstehend definierte Bedeutung haben), wobei eine Meldrum-Säure der Formel (XV) 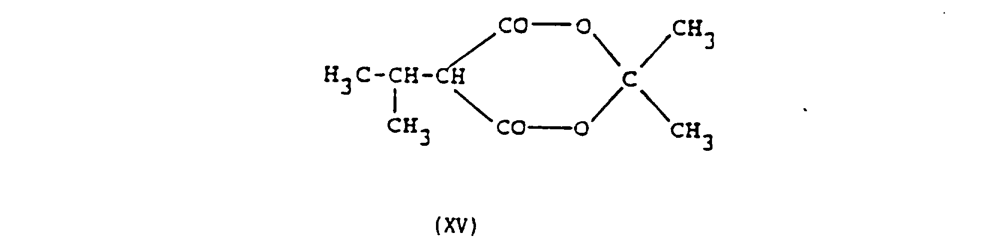 mit einem in geeigneter Weise geschützten Tyrosin der Formel (XVI) 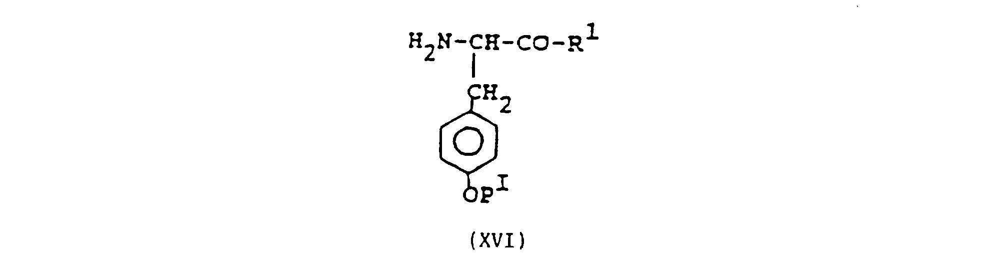
(worin alle Symbole die vorstehend definierte Bedeutung haben), in Gegenwart eines Silanisierungsmittels kondensiert wird.

12. Verfahren nach den Ansprüchen 6-11, worin:
P^{L} eine tert.-Butoxycarbonyl- oder eine tert.-Amyloxycarbonylgruppe bedeutet,
P^{G} eine Benzylsulfonylgruppe bedeutet, die ggf. substituiert ist,
P eine tert.-Butyl-, tert.-Amyl-, Benzyl- oder substituierte Benzylgruppe bedeutet und
P^{I} eine tert.-Butyl- oder eine tert.-Amylgruppe bedeutet.

13. Verfahren zur Herstellung einer pharmazeutischen Verbindung, wobei ein Wirkstoff, wie er im Verfahren nach Anspruch 1 erhalten worden ist, mit einem pharmazeutisch verträglichen inerten Träger und ggf. mit anderen standardmäßigen Additiven, die in geeigneter Weise ausgewählt werden, vereinigt wird.

## Revendications (Revendications pour l'(les) Etat(s) contractant(s) suivant(s): AT, BE, CH, DE, DK, FR, GB, LI, LU, NL, SE)

1. Analogue de thymopentine représenté par l'une des formules suivantes : 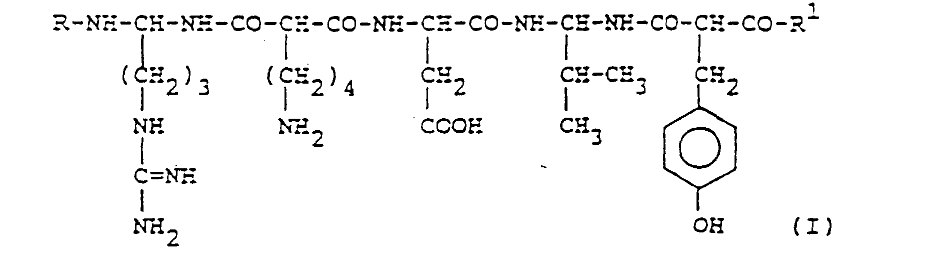 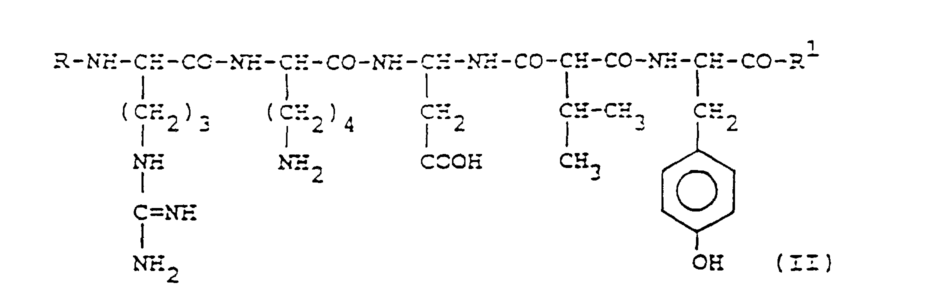 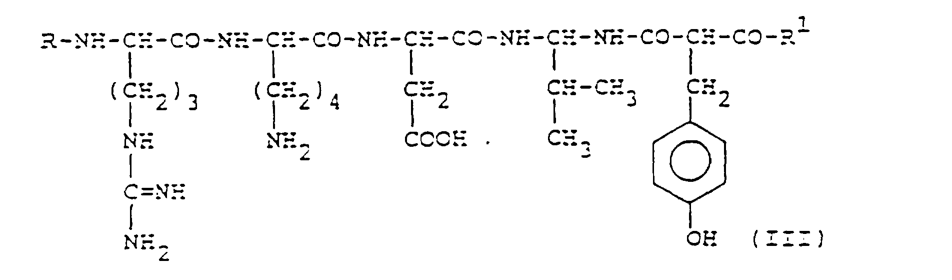 dans lesquelles :
R est un atome d'hydrogène ou un groupe acyle labile par voie métabolique ou un groupe acyle dont la liaison avec le groupe amino est rapidement brisée dans les premières étapes du chemin métabolique du produit, et
R¹ est un groupe de formule -OR, dans laquelle R est un atome d'hydrogène ou un groupe alkyle ayant une chaîne droite ou ramifiée contenant de 1 à 6 atomes de carbone, un groupe alcényle ou un groupe alcynyle ayant une chaîne droite ou ramifiée contenant de 3 à 6 atomes de carbone, ou un groupe aryl-alkyle ou alkyl-aryle contenant de 7 à 12 atomes de carbone,
et les sels d'addition d'acide ou de base correspondants acceptables du point de vue pharmaceutique.

2. Composé suivant la revendication 1, dans lequel R et R¹ sont tels que définis dans la revendication 1 et R est un atome d'hydrogène.

3. Composé suivant la revendication 2, dans lequel R est un atome d'hydrogène.

4. Composé suivant la revendication 3, qui est choisi parmi :
[gArg¹, (R,S)mLys, gVal⁴, (R,S)mTyr⁵] TP5,
[gAsp³, (R,S)mVal⁴] TP5, [(R,S) mVal⁴, gTyr⁵] TP5,
et leurs sels d'addition.

5. Composé suivant la revendication 4, qui est choisi parmi :
[gArg¹, (R,S) mLys, gVal⁴, (R,S) mTyr⁵] TP5,
et ses sels d'addition.

6. Composition pharmaceutique comprenant un composé suivant la revendication 1 en tant que composant actif.

7. Composé suivant la revendication 1, pour l'utilisation en tant que médicament.

8. Procédé pour la préparation d'un analogue de thymopentine représenté par l'une des formules générales suivantes : 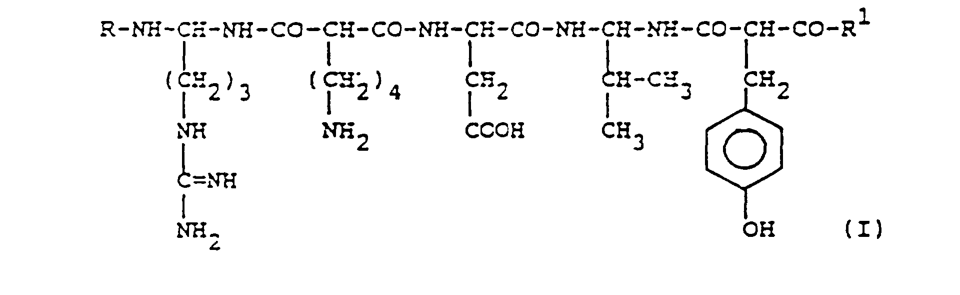 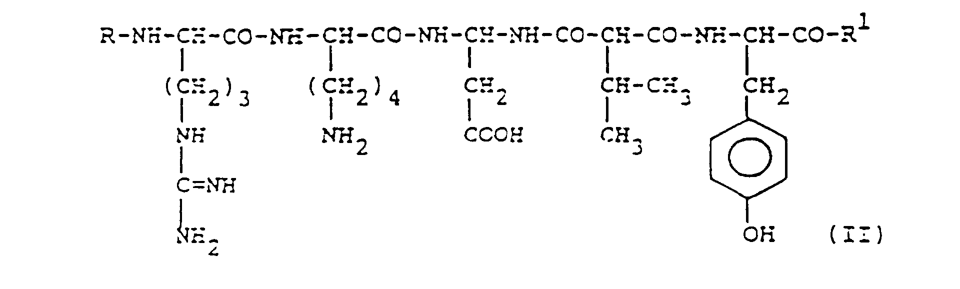 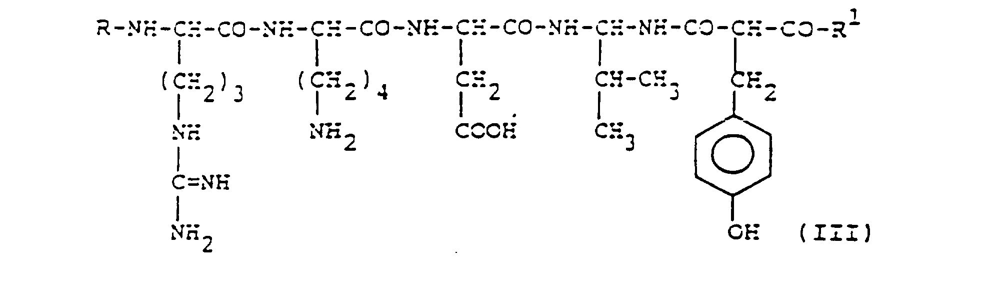 dans lesquelles :
R est un atome d'hydrogène ou un groupe acyle, et
R¹ est un groupe de formule -OR, dans laquelle R est un atome d'hydrogène ou un groupe alkyle ayant une chaîne droite ou ramifiée contenant de 1 à 6 atomes de carbone, un groupe alcényle ou un groupe alcynyle ayant une chaîne droite ou ramifiée contenant de 3 à 6 atomes de carbone, ou un groupe aryl-alkyle ou alkyl-aryle contenant de 7 à 12 atomes de carbone,
et des sels d'addition d'acide ou de base correspondants acceptables du point de vue pharmaceutique,
ce procédé consistant à éliminer les groupes protecteurs des composés correspondants des formules (Ia), (IIa) et (IIIa) : 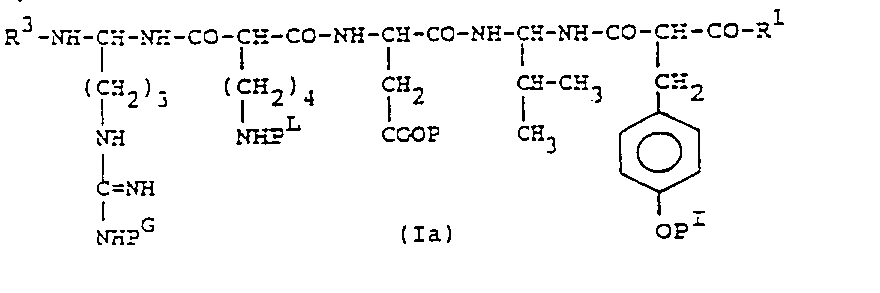 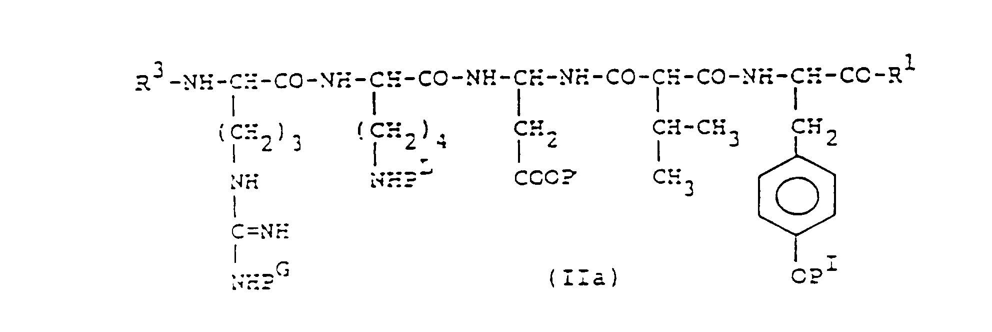 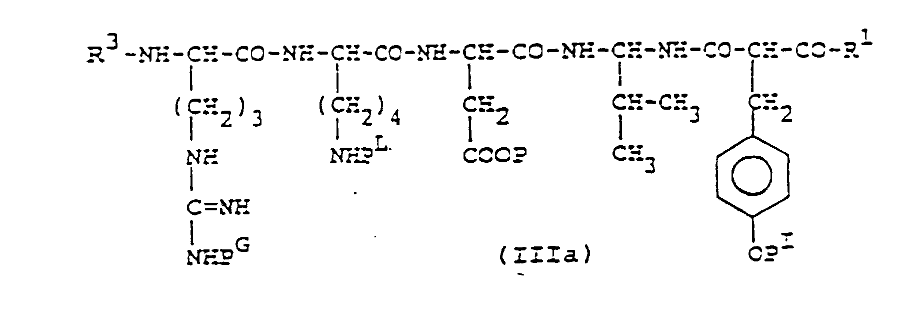 dans lesquelles :
R³ est R ou un groupe de protection pour la fonction alpha-amino,
P^{G} a la signification d'un groupe protecteur convenable pour la fonction guanidine,
P^{L} a la signification d'un groupe protecteur pour la fonction amino dans la chaîne latérale,
P est un groupe protecteur pour la fonction carboxy,
P^{I} est un groupe protecteur pour la fonction hydroxy de la tyrosine, et
R¹ est tel que défini plus haut.

9. Composé intermédiaire représenté par l'une des formules (Ia), (IIa), (IIIa), (IV), (VI), (VII), (VIII) , (IX), (X) et (XIV) : 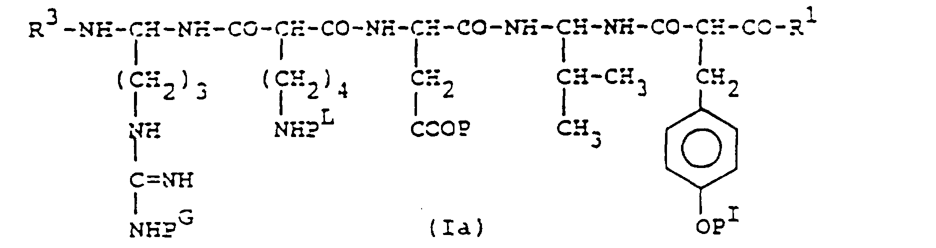 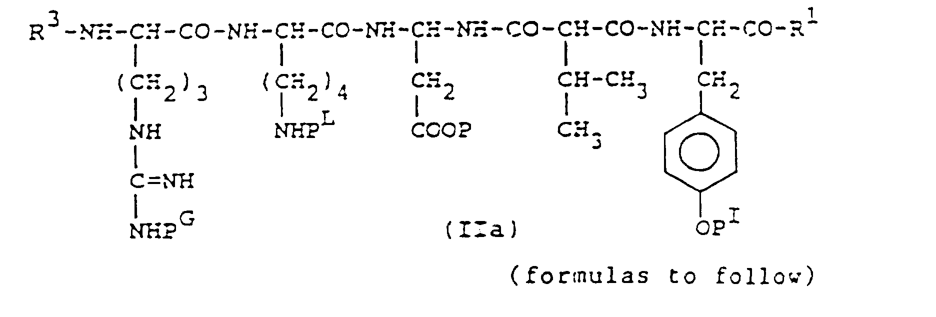 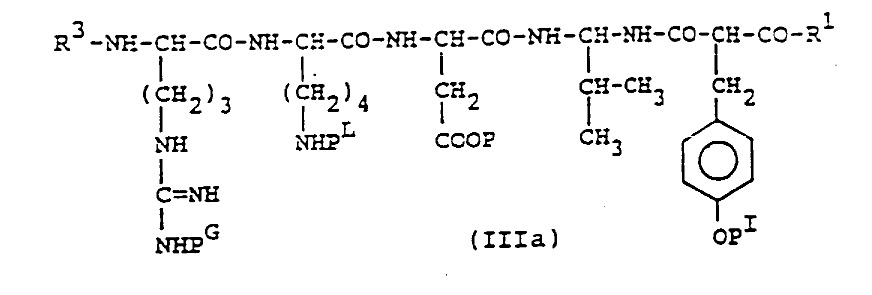 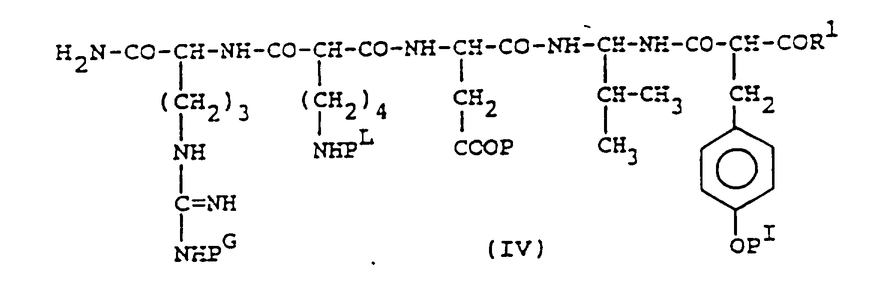 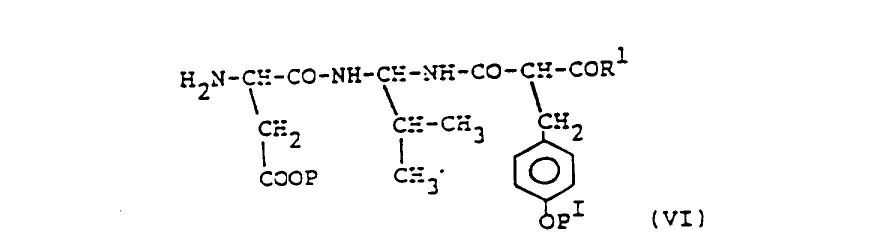 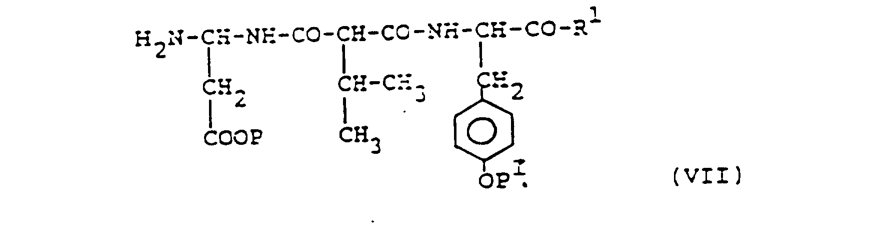 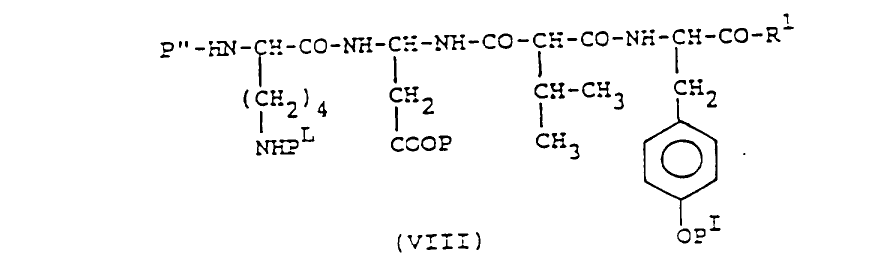 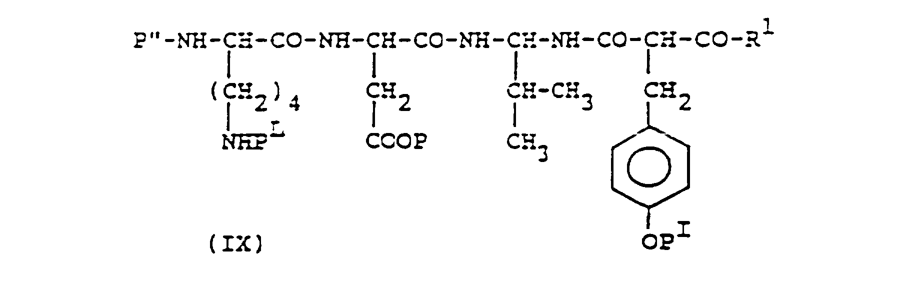 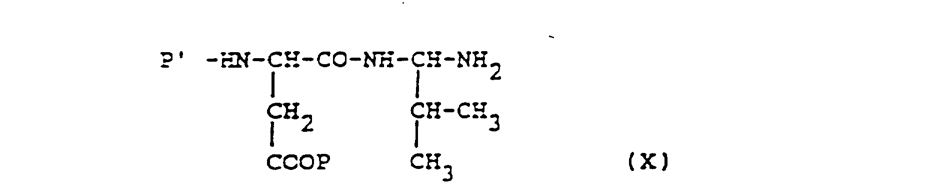 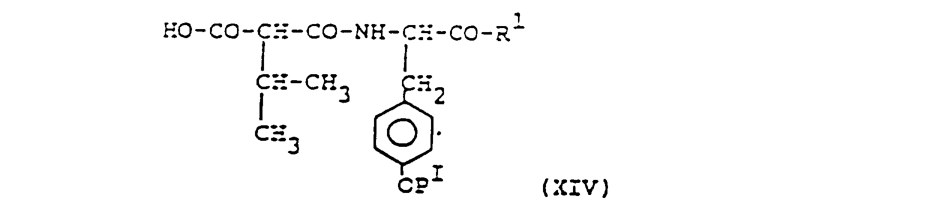 dans lesquelles :
P" est un atome d'hydrogène ou un groupe protecteur convenable pour la fonction alpha-amino,
P^{G} a la signification d'un groupe protecteur convenable pour la fonction guanidine,
P^{L} a la signification d'un groupe protecteur pour la fonction amino dans la chaîne latérale,
P est un groupe protecteur pour la fonction carboxy,
R¹ est un groupe de formule -OR dans laquelle R est tel que défini plus haut, et
P^{I} est un groupe protecteur pour la fonction hydroxy de la tyrosine.

10. Composé intermédiaire représenté par l'une des formules (Ia) , (IIa), (IIIa), (IV) , (VI), (VII), (VIII), (IX), (X) et (XIV) de la revendication 9, dans lequel :
P^{L} est un groupe t-butoxycarbonyle ou un groupe t-amyloxycarbonyle,
P^{G} est un groupe benzènesulfonyle éventuellement substitué,
P est un groupe t-butyle, un groupe t-amyle, un groupe benzyle ou un groupe benzyle substitué, et
P^{I} est un groupe t-butyle ou un groupe t-amyle.

## Revendications (Revendications pour l'(les) Etat(s) contractant(s) suivant(s): ES, GR)

1. Procédé pour la préparation d'un analogue de thymopentine représenté par l'une des formules générales suivantes : 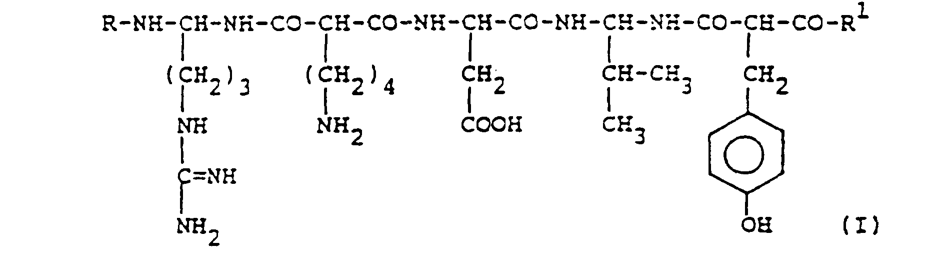 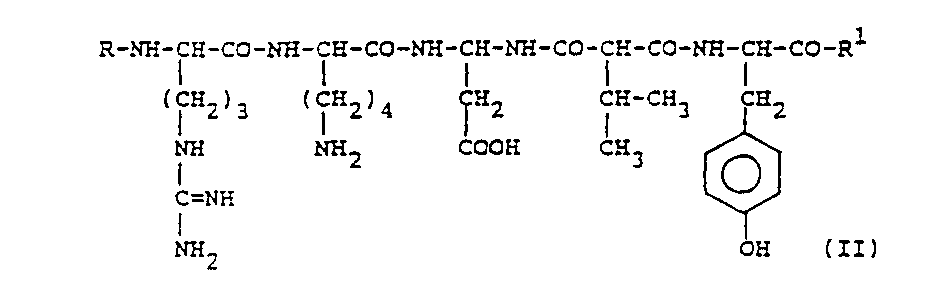 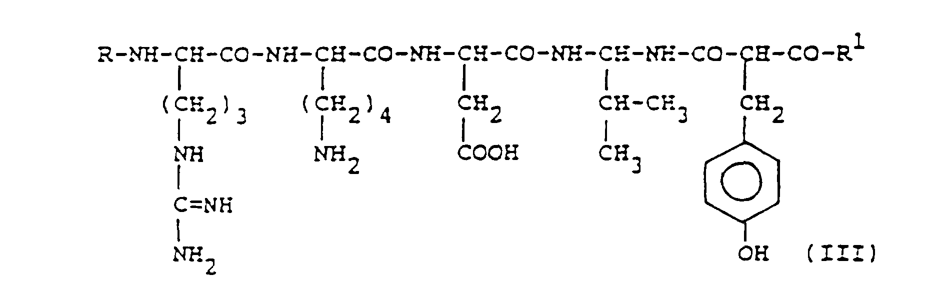 dans lesquelles :
R est un atome d'hydrogène ou un groupe acyle labile par voie métabolique ou un groupe acyle dont la liaison avec le groupe amino est rapidement brisée dans les premières étapes du chemin métabolique du produit, et
R¹ est un groupe de formule -OR, dans laquelle R est un atome d'hydrogène ou un groupe alkyle ayant une chaîne droite ou ramifiée contenant de 1 à 6 atomes de carbone, un groupe alcényle ou un groupe alcynyle ayant une chaîne droite ou ramifiée contenant de 3 à 6 atomes de carbone, ou un groupe aryl-alkyle ou alkyl-aryle contenant de 7 à 12 atomes de carbone,
et des sels d'addition d'acide ou de base correspondants acceptables du point de vue pharmaceutique,
ce procédé consistant à éliminer les groupes protecteurs des composés correspondants des formules (Ia), (IIa) et (IIIa) : 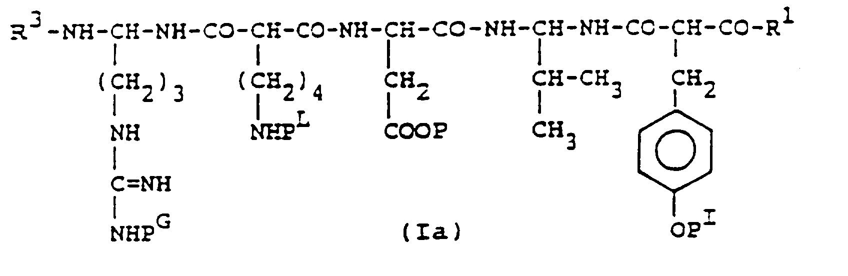 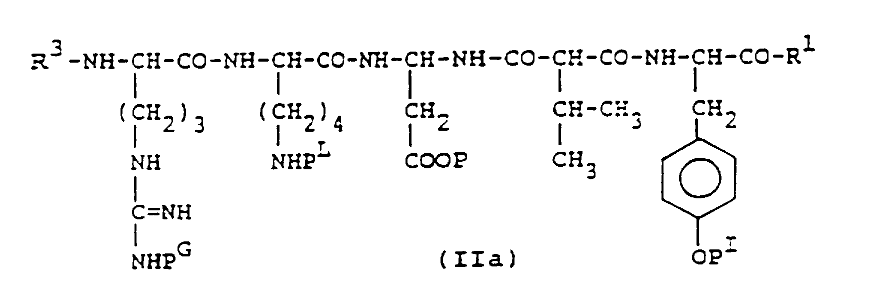 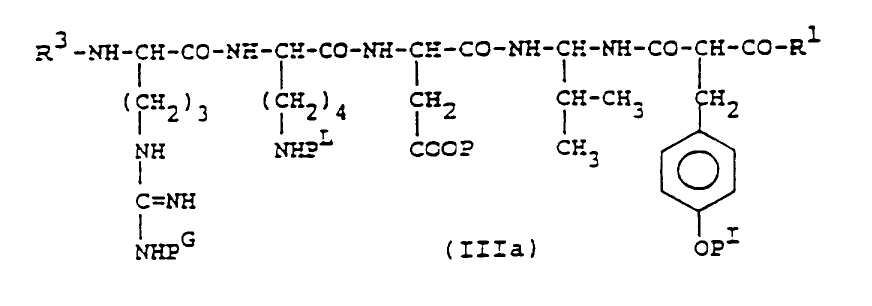 dans lesquelles :
R³ est R ou un groupe de protection pour la fonction alpha-amino,
P^{G} a la signification d'un groupe protecteur convenable pour la fonction guanidine,
P^{L} a la signification d'un groupe protecteur pour la fonction amino dans la chaîne latérale,
P est un groupe protecteur pour la fonction carboxy,
P^{I} est un groupe protecteur pour la fonction hydroxy de la tyrosine, et
R¹ est tel que défini plus haut.

2. Procédé suivant la revendication 1, dans lequel R et R¹ sont tels que définis dans la revendication 1 et R est un atome d'hydrogène.

3. Procédé suivant la revendication 2, dans lequel R est un atome d'hydrogène.

4. Procédé suivant la revendication 3, dans lequel le composé final est choisi parmi :
[gArg¹, (R,S)mLys, gVal⁴, (R,S) mTyr⁵] TP5,
[gAsp³, (R,S)mVal⁴] TP5, [(R,S) mVal⁴, gTyr⁵] TP5,
et leurs sels d'addition.

5. Procédé suivant la revendication 4, dans lequel le composé final est :
[gArg¹, (R,S)mLys, gVal⁴, (R,S) mTyr⁵] TP5,
et ses sels d'addition.

6. Procédé pour la préparation d'un composé de formule (Ia) : 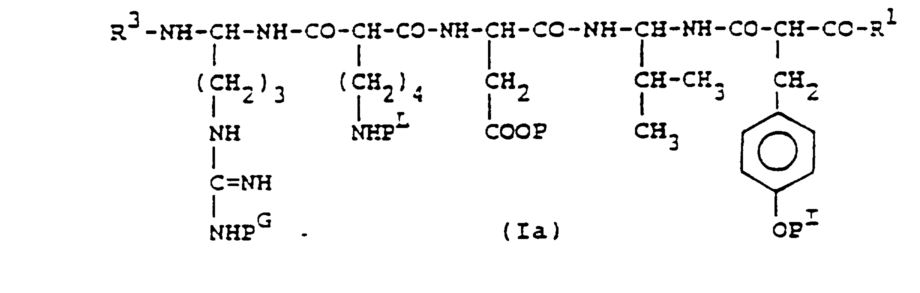 (dans laquelle R³ = R et les autres symboles sont tels que définis plus haut), dans lequel un composé de formule (IV) : 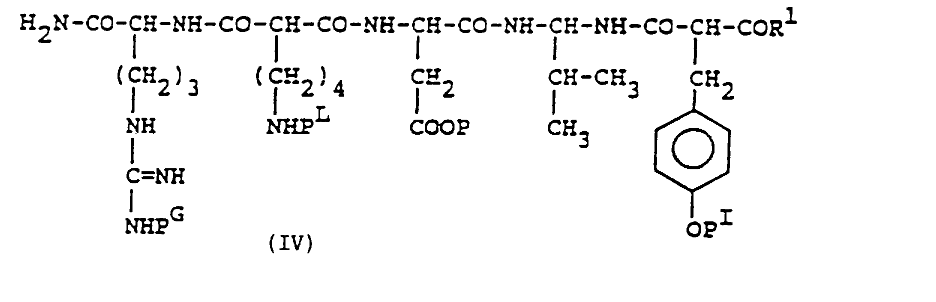 (dans laquelle tous les symboles sont tels que définis plus haut) est traité avec du 1,1-bis-trifluoroacétoxyiodobenzène, puis la fonction amino terminale ainsi formée est soumise à une acylation.

7. Procédé pour la préparation d'un composé de formule (IV) : 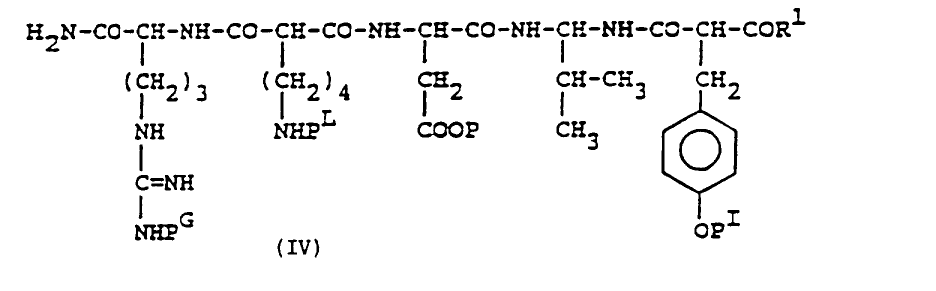 (dans laquelle tous les symboles sont tels que définis plus haut), dans lequel un composé de formule (V) : 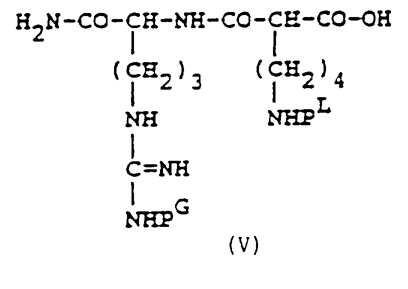 (dans laquelle tous les symboles sont tels que définis plus haut), est condensé avec un composé de formule (VI) : 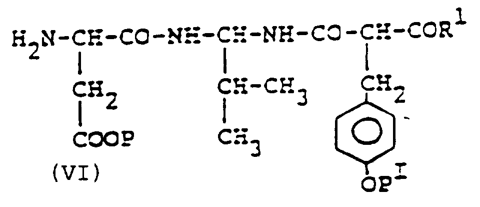 (dans laquelle tous les symboles sont tels que définis plus haut).

8. Procédé pour la préparation de composés de formules (IIa) et (IIIa) : 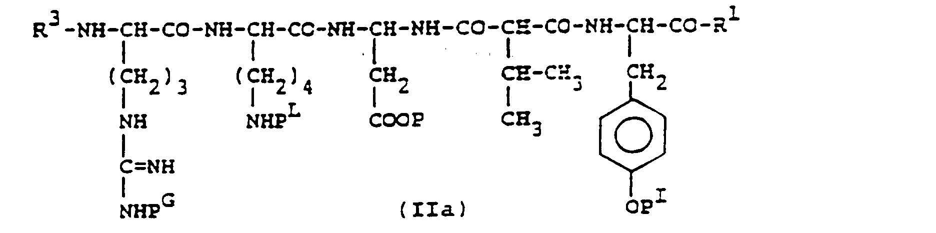 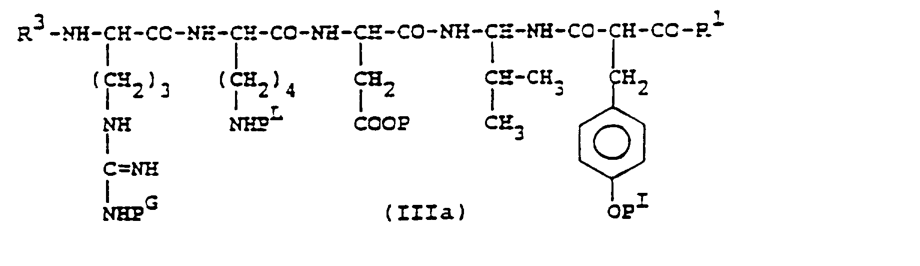 (dans lesquelles tous les symboles sont tels que définis plus haut), dans lequel des composés de formules (VII) et (VI) : 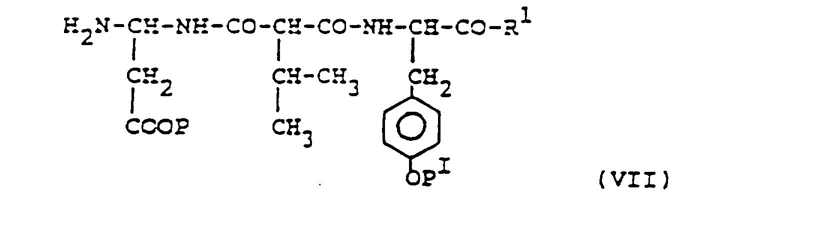 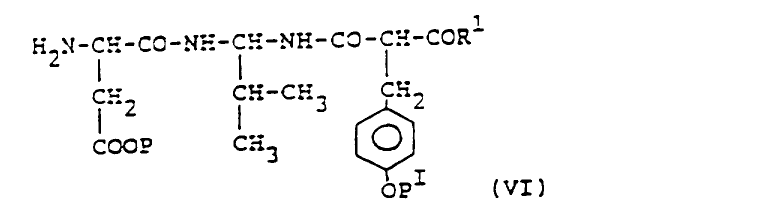 (dans laquelle tous les symboles sont tels que définis plus haut), sont successivement condensés avec un résidu lysine ou arginine pour donner les composés correspondants de formules (VIII) et (IX): 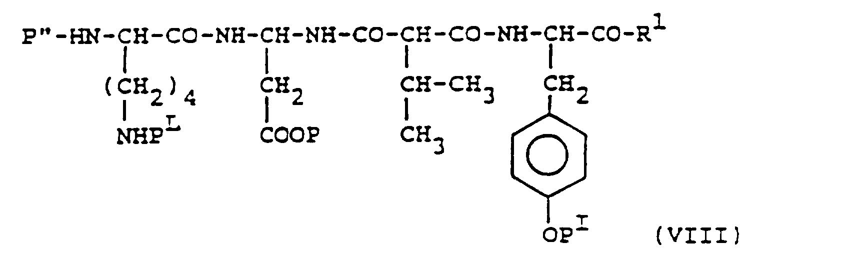 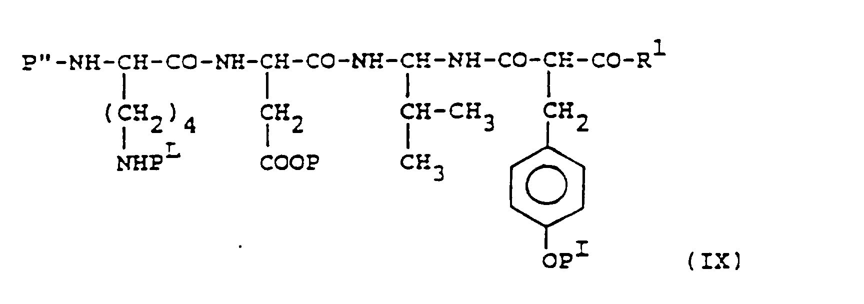 (dans laquelle P" est un atome d'hydrogène ou un groupe protecteur pour la fonction alpha amino, qui est être facilement éliminé dans des conditions qui n'engendrent pas l'élimination des autres groupes protecteurs et les autres symboles sont tels que définis plus haut).

9. Procédé pour la préparation d'un composé de formule (VI) : 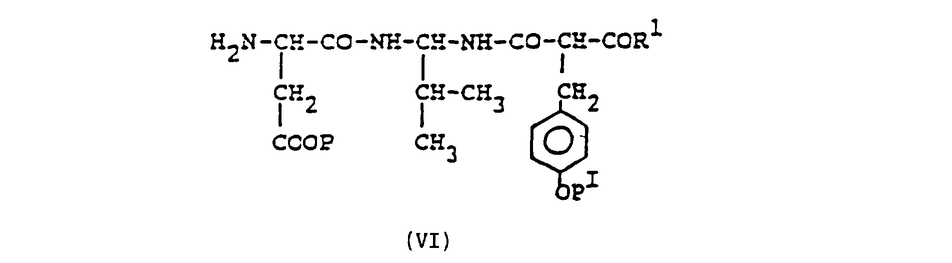 (dans laquelle tous les symboles sont tels que définis plus haut), dans lequel un dipeptide de formule (X) : 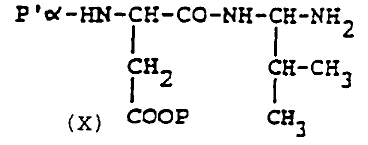 (dans laquelle P est un groupe protecteur pour la fonction carboxy et P' est un groupe protecteur pour la fonction amino en position alpha),
est condensé avec un composé de formule (XI) : 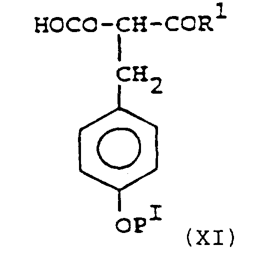 dans laquelle R¹ et P¹ sont tels que définis plus haut.

10. Procédé pour la préparation d'un composé de formule (VIII) : 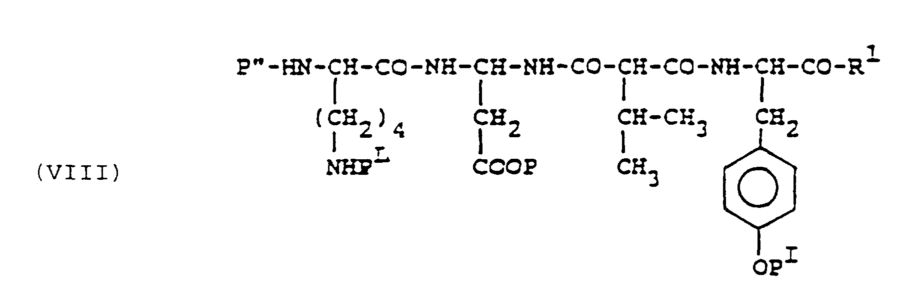 (dans laquelle tous les symboles sont tels que définis plus haut), dans lequel un amide de formule (XIII) : 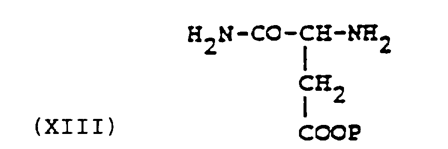 (dans laquelle tous les symboles sont tels que définis plus haut) est condensé avec un composé de formule (XIV) : 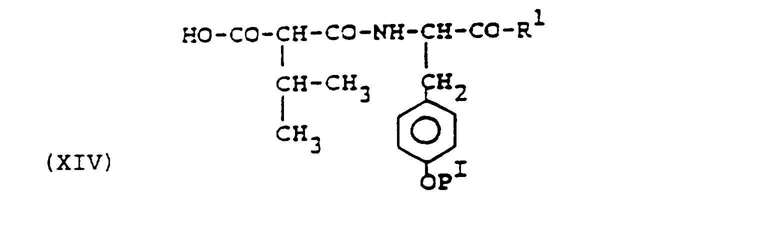 (dans laquelle tous les symboles sont tels que définis plus haut), puis le groupe amide est converti en groupe amino primaire par traitement avec du TIB.

11. Procédé pour la préparation d'un composé de formule (XIV) : 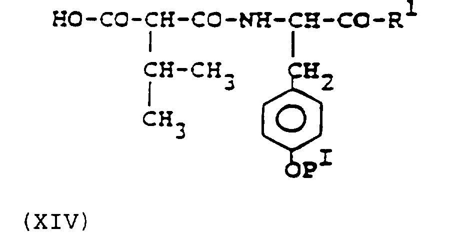 (dans laquelle tous les symboles sont tels que définis plus haut), dans lequel un acide de Meldrum de formule (XV) : 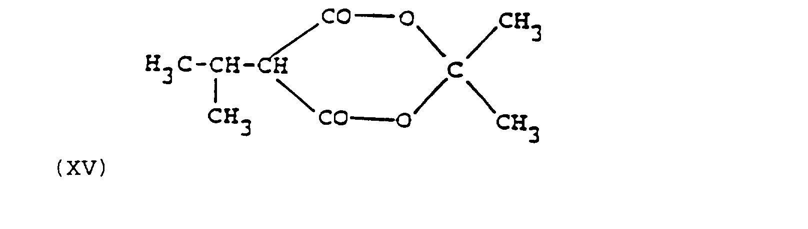 est condensé avec une tyrosine protégée de façon appropriée de formule (XVI) : 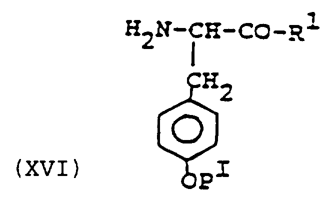 (dans laquelle tous les symboles sont tels que définis plus haut) en présence d'un agent silanisant.

12. Procédé suivant les revendications 6 à 11, dans lequel:
P^{L} est un groupe t-butoxycarbonyle ou un groupe t-amyloxycarbonyle,
P^{G} est un groupe benzènesulfonyle éventuellement substitué,
P est un groupe t-butyle, un groupe t-amyle, un groupe benzyle ou un groupe benzyle substitué, et
P^{I} est un groupe t-butyle ou un groupe t-amyle.

13. Procédé pour la préparation d'une composition pharmaceutique, dans lequel un composé actif tel qu'il est obtenu suivant la revendication 1, est combiné avec un véhicule inerte acceptable du point de vue pharmaceutique et éventuellement, avec certains additifs standard choisis de façon appropriée.
